(19) Europäisches Patentamt
European Patent Office
Office européen des brevets

(11) EP 0 816 496 A2

(12) EUROPÄISCHE PATENTANMELDUNG

(43) Veröffentlichungstag:
07.01.1998 Patentblatt 1998/02

(51) Int. Cl.⁶: **C12N 15/12**, C07K 14/725,
G01N 33/68, C12N 5/08,
A61K 31/70, A61K 38/17,
C07K 16/30, C07K 19/00,
A01K 67/027

(21) Anmeldenummer: 97110231.4

(22) Anmeldetag: 23.06.1997

(84) Benannte Vertragsstaaten:
AT BE CH DE DK ES FI FR GB GR IE IT LI LU MC
NL PT SE

(30) Priorität: 24.06.1996 DE 19625191

(71) Anmelder:
BOEHRINGER MANNHEIM GMBH
68305 Mannheim (DE)

(72) Erfinder:
Schendel, Dolores J., Prof. Dr.
80469 München (DE)

(74) Vertreter:
Weiss, Wolfgang, Dipl.-Chem. Dr. et al
Patentanwälte
Weickmann & Partner,
Kopernikusstrasse 9
81679 München (DE)

(54) **Nierenkarzinom-spezifische-T-Zellen**

(57) Die vorliegende Erfindung betrifft neue Nucleinsäure- und Aminosäuresequenzen des humanen T-Zellrezeptors und deren Verwendung für die Diagnostik und Therapie von Karzinomen, insbesondere Nierenzellkarzinomen.

EP 0 816 496 A2

**Beschreibung**

Die vorliegende Erfindung betrifft neue Nucleinsäure- und Aminosäuresequenzen des humanen T-Zellrezeptors und deren Verwendung für die Diagnostik und Therapie von Karzinomen, insbesondere Nierenzellkarzinomen.

Die T-Lymphozyten des Immunsystems sind für die zelluläre Immunantwort verantwortlich. Sie sind zur Erkennung und Beseitigung von erkrankten Körperzellen in der Lage, z.B. von Zellen, die fremde Proteine enthalten, oder von Tumorzellen. Die Erkennung erkrankter Körperzellen erfolgt durch den sogenannten T-Zellrezeptor (TCR), der ein für die erkrankte Zelle spezifisches Antigen in Form von kurzen Peptidfragmenten bindet. Diese Peptidfragmente werden von MHC-Molekülen an der Zelloberfläche präsentiert.

T-Zellrezeptoren bestehen aus zwei verschiedenen Polypeptiduntereinheiten, üblicherweise den sogenannten T-Zellrezeptor $\alpha$- oder $\beta$-Ketten, die miteinander durch eine Disulfidbrücke verbunden sind. Die $\alpha$- und $\beta$-Ketten sind wiederum aus variablen und konstanten Regionen zusammengesetzt. Die variablen Regionen der $\alpha$-Kette umfassen V- und J-Gensegmente und die variablen Regionen der $\beta$-Kette umfassen V-, D- und J-Gensegmente.

Das TCR-$\alpha$-Kettengen besteht aus über 100 variablen Segmenten, von denen jedes ein Exon für eine V-Region enthält, dem ein anderes Exon voransteht, das für eine Leadersequenz kodiert, die einen Transport des Proteins an die Zelloberfläche ermöglicht. Eine Gruppe von 61 J-Segmenten liegt in beträchtlicher Entfernung von den V-Segmenten. Den J-Segmenten folgt ein einzelnes C-Segment für den konstanten Bereich, das wiederum getrennte Exons für die konstante Region und die Hinge-Region sowie ein Exon für die Transmembran- und Cytoplasmaregionen enthält.

Das TCR-$\beta$-Kettengen enthält eine Gruppe von ungefähr 30 V-Gensegmenten, die in einiger Entfernung von 2 getrennten Clustern liegen, die jeweils ein einzelnes D-Segment und 6 bzw. 7 J-Segmente sowie ein einzelnes C-Segment enthalten. Jedes konstante Segment der $\beta$-Kette besitzt separate Exons für die konstante, die Hinge-, die Transmembran- und die Cytoplasmaregion.

Während der Entwicklung der T-Zelle werden die getrennten Segmente durch somatische Rekombination verknüpft. Für die $\alpha$-Kette gelangt ein V$\alpha$-Gensegment neben ein J$\alpha$-Gensegment und damit entsteht ein funktionelles Exon. Durch Transkription und Splicing des VJ$\alpha$-Exon an die konstante Region wird die mRNA gebildet, die zur TCR-$\alpha$-Kette translatiert wird. Die Umordnung der für die variable Domäne der $\beta$-Kette kodierenden Gensegmente V$\beta$, D$\beta$ und J$\beta$ schafft ein funktionelles Exon, das transkribiert und durch Splicing an C$\beta$ angefügt wird. Die entstandene mRNA wird zur TCR-$\beta$-Kette translatiert. Die $\alpha$- und $\beta$-Ketten verbinden sich nach ihrer Biosynthese zum $\alpha{:}\beta$ TCR-Heterodimer. Der für die Spezifität der Antigenerkennung verantwortliche hochvariable Bereich des TCR, der im Bereich der Verknüpfung von V-, (D-) und J-Gensegmenten liegt, wird als CDR3-Region bezeichnet.

Aufgrund der hohen Variabilität von T-Zellrezeptoren ist die Identifizierung spezifischer Nucleotid- und Aminosäuresequenzen insbesondere im Bereich der CDR3-Antigenerkennungsregion nur mit sehr hohem Aufwand möglich. Es besteht daher ein großes Bedürfnis, Nucleinsäure- und Aminosäuresequenzen von T-Zellrezeptoren bereitzustellen, die spezifisch zur Erkennung von klinisch relevanten Peptidantigenen, insbesondere von tumorspezifischen Peptidantigenen in der Lage sind.

Erfindungsgemäß konnten Tumor-infiltrierende Lymphozyten (TIL) aus einem Nierenkarzinom gewonnen werden, die eine hohe Spezifität für Tumorgewebe aus Patienten mit dem HLA-A∗0201-Allel besitzen. Mit gesundem Nierengewebe aus dem gleichen Patienten zeigen diese TIL keine Reaktion.

Es wurde eine Analyse der Nucleotid- und Aminosäuresequenzen der von diesen TIL exprimierten T-Zellrezeptoren durchgeführt. Dabei wurde zunächst nach Kultivierung und periodischer Restimulierung der TIL über eine Dauer von 62 bzw. 74 Tagen ein einheitlicher CD8$^+$ T-Zellklon erhalten. Die für die $\alpha$- bzw. $\beta$-Kette des T-Zellrezeptors kodierende cDNA wurde sequenziert. Die Nucleotid- und Aminosäuresequenz der $\alpha$-Kette sind in den Sequenzprotokollen SEQ ID No. 1 und SEQ ID No. 2 angegeben. Die CDR3$\alpha$-Region in SEQ ID No. 1 reicht von bp 313 bis 348 entsprechend den Aminosäuren 87-98 in SEQ ID No. 2. Die Nucleotid- und Aminosäuresequenz der $\beta$-Kette sind in den Sequenzprotokollen SEQ ID No. 3 und SEQ ID No. 4 angegeben. Die CDR3$\beta$-Region in SEQ ID No. 3 reicht von bp 331 bis 369 in SEQ ID No. 3 entsprechend den Aminosäuren 90-102.

Bei der $\alpha$-Kette wurde in der variablen Region eine Kombination von V$\alpha$20 mit J$\alpha$22 und bei der $\beta$-Kette eine Kombination von V$\beta$22, D$\beta$2 und J$\beta$2.7 gefunden.

Anschließend wurde eine Sequenzanalyse der tumorspezifischen T-Zellrezeptoren bei einer nur 24-tägigen Kultivierung durchgeführt. Dabei wurde kein einheitlicher T-Zellklon, sondern ein Gemisch aus mehreren T-Zellspezies gefunden. Für die $\alpha$-Kette konnten die in SEQ ID No. 2 angegebene Aminosäuresequenz sowie insgesamt zwei weitere Aminosäuresequenzen identifiziert werden. 11 von 56 untersuchten T-Zellspezies kodierten dabei für die in SEQ ID No. 2 von Position 87 bis 98 angegebene Aminosäuresequenz der CDR3$\alpha$-Region. Die Nucleotidsequenz der $\alpha$-Ketten in diesen T-Zellen unterschied sich von der in SEQ ID No. 1 angegebenen Sequenz nur durch einen Austausch T gegen G an Position 324.

Die Nucleotid- und Aminosäuresequenz der CDR3-Region einer weiteren $\alpha$-Kette, die in 38 der 56 untersuchten T-Zellen identifiziert wurde, ist in den Sequenzprotokollen SEQ ID No. 5 und 6 angegeben. Darüber hinaus wurden zwei weitere T-Zellspezies identifiziert, die eine CDR3$\alpha$-Region mit derselben Aminosäuresequenz wie in SEQ ID No. 6 gezeigt enthalten, die sich aber jeweils durch einen Basenaustausch in der Nucleotidsequenz (C an Position 9 durch G bzw. T an Position 12 durch C) unterscheiden.

Die Nucleotid- und Aminosäuresequenz der CDR3α-Region aus einer dritten T-Zellvariante, die in einer Häufigkeit von 5 von 56 untersuchten T-Zellspezies auftrat, ist in den Sequenzprotokollen SEQ ID No. 7 und 8 angegeben.

Die entsprechende Sequenzierung der β-Ketten ergab insgesamt 6 unterschiedliche Aminosäuresequenzen für die CDR3-Region. Eine CDR3β-Sequenz, die bei 15 von 50 untersuchten T-Zellen gefunden wurde, ist in den Sequenzprotokollen SEQ ID No. 9 und 10 angegeben. Eine weitere T-Zellspezies enthielt die gleiche Aminosäuresequenz, aber eine unterschiedliche Nucleotidsequenz (Austausch von A an Position 15 durch T).

Jeweils eine T-Zellspezies enthielt die in den Sequenzprotokollen SEQ ID No. 11 und 12, 13 und 14 bzw. 15 und 16 angegebenen Nucleotid- bzw. Aminosäuresequenzen in der CDR3β-Region.

27 von 50 Klonen enthielten die in den Sequenzprotokollen 17 und 18 gezeigten Nucleotid- und Aminosäuresequenzen in der CDR3β-Region. 4 von 50 untersuchten Klonen enthielten die in den Sequenzprotokollen SEQ ID. No. 19 und 20 angegebenen Nucleotid- bzw. Aminosäuresequenzen in der CDR3β-Region.

Weiterhin wurde eine in situ-Sequenzierung von TIL, d.h. eine Sequenzierung ohne vorherige Kultivierung, durchgeführt. Hierzu wurde die Gesamt-RNA aus dem Tumor isoliert, mit einem TCRα- bzw. TCRβ-spezifischen Primer und reverser Transkriptase eine TCR-spezifische cDNA hergestellt und diese cDNA unter Verwendung familienspezifischer Primer (Vα20 bzw. Vβ22) selektiv durch PCR amplifiziert. Die Amplifikationsprodukte wurden in E.coli kloniert und sequenziert. Dabei wurde eine Reihe von Einzelsequenzen erhalten.

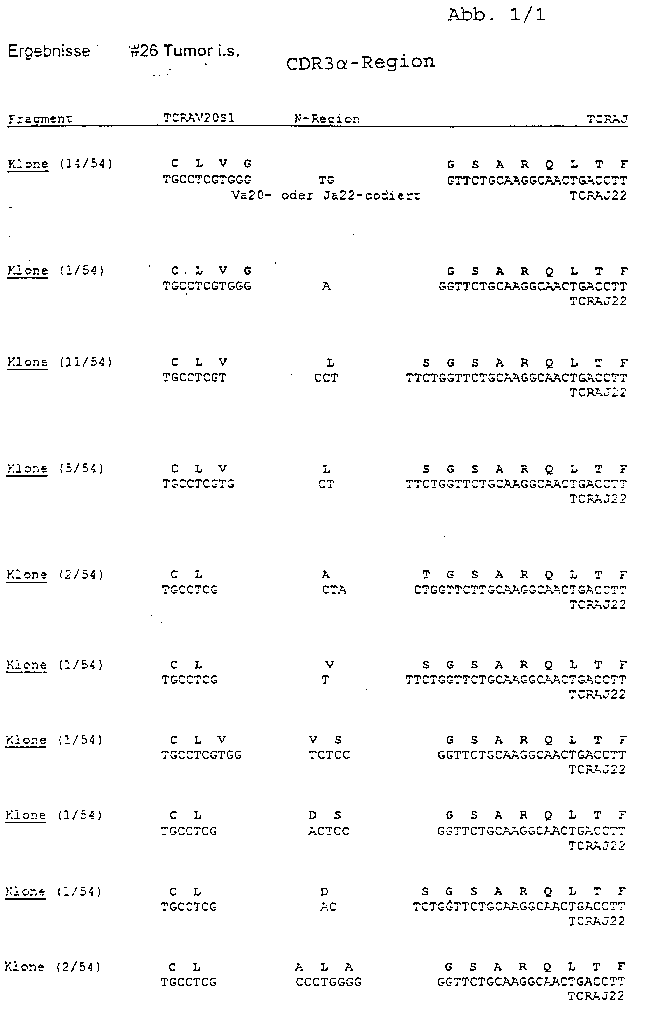

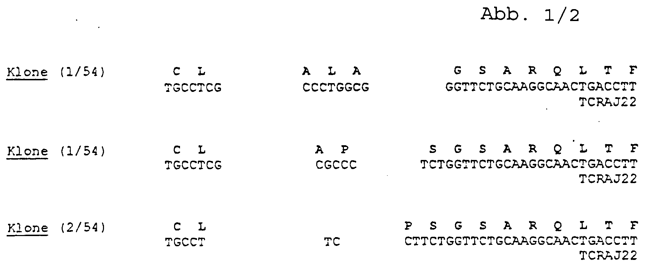

Circa 60 % aller Sequenzen der α-Kette entsprechen den in den Sequenzprotokollen SEQ ID No. 2, 6 und 8 angegebenen Aminosäuresequenzen. Weitere 20 % hatten sehr ähnliche Sequenzen, die ebenfalls aus einer Kombination von Vα20 und Jα22 bestehen. Eine Übersicht der bei dieser in situ-Sequenzierung von T-Zellen des Patienten 26 identifizierten CDR3α-Regionen ist in Abb. 1 gezeigt.

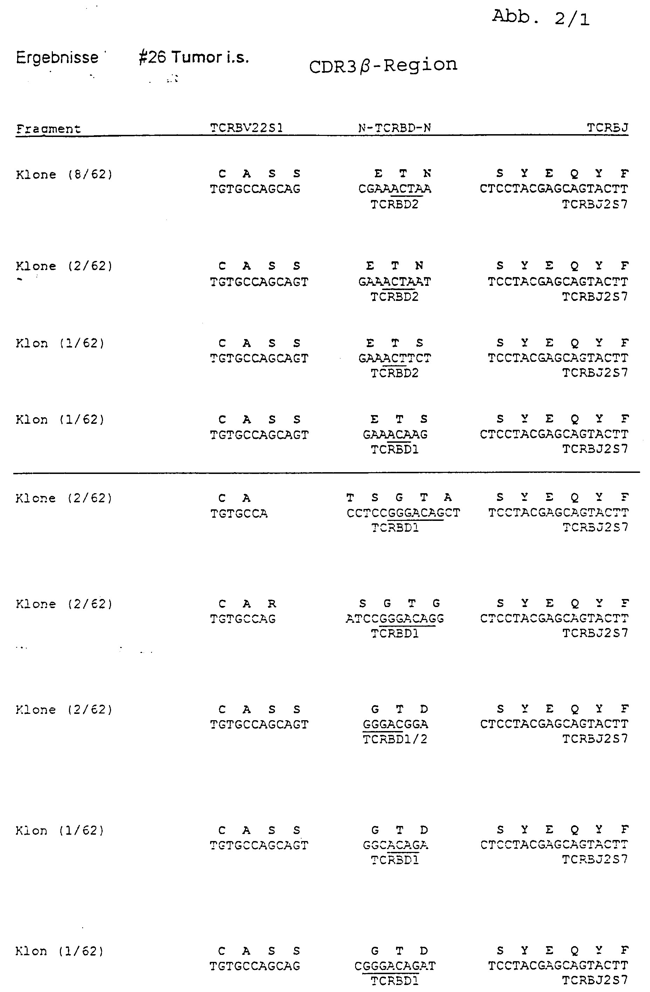

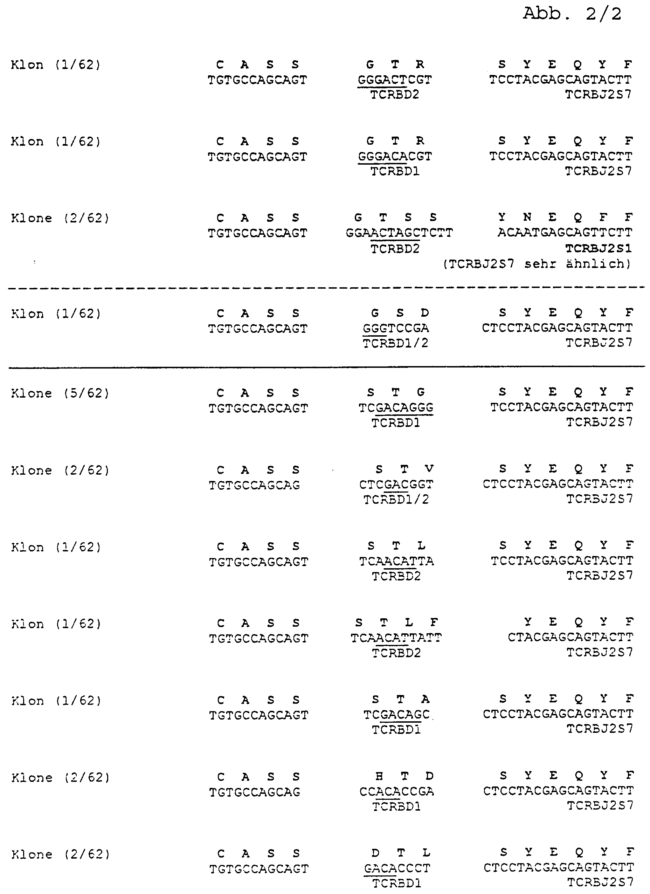

Weiterhin wurde bei der in situ-Sequenzierung gefunden, daß ca. 70 % aller Sequenzen der β-Kette den in den Sequenzprotokollen 4, 10, 12, 14, 16, 18 und 20 angegebenen Aminosäuresequenzen entsprechen. Eine Übersicht der bei der in situ-Sequenzierung identifizierten CDR3-Sequenzen der β-Kette ist in Abb. 2 gezeigt.

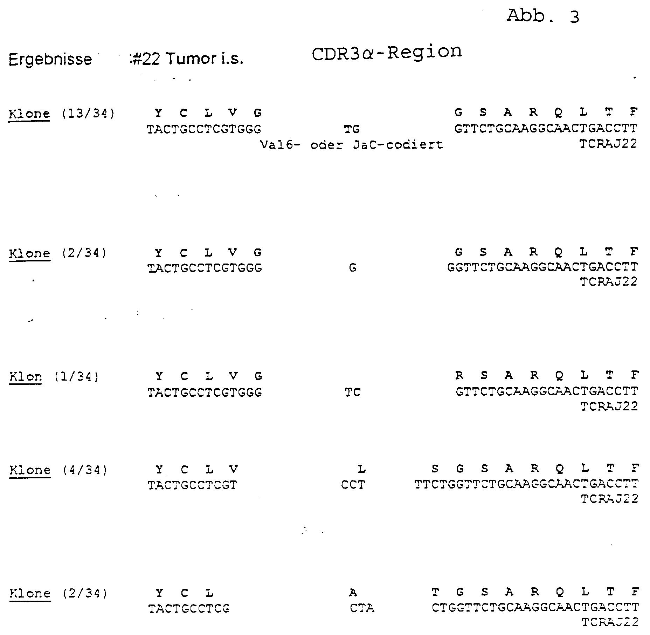

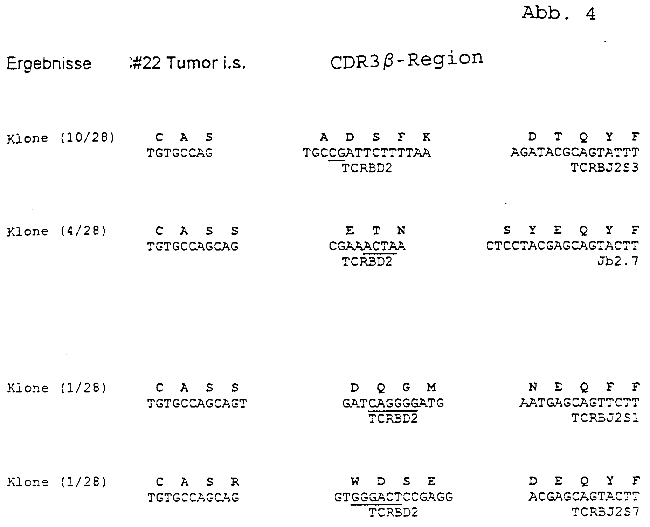

In einem Kontrollexperiment wurden TIL aus einem anderen Patienten mit dem HLA-A*0201-Allel durch in situ-Sequenzierung analysiert. Dabei wurde festgestellt, daß die CDR3-α-Regionen von 15 bzw. 4 der insgesamt untersuchten 34 T-Zellspezies die in SEQ ID No. 2 bzw. SEQ ID No. 6 dargestellte Aminosäuresequenzen enthielten. Eine Übersicht der relevanten CDR3α-Sequenzen und ihre Häufigkeit ist in Abb. 3 gezeigt. Eine Übersicht der Ergebnisse, die bei der Sequenzierung von CDR3-Regionen der β-Kette erhalten wurden, ist in Abb. 4 dargestellt.

In einem ersten Aspekt betrifft die vorliegende Erfindung somit eine Nucleinsäure, die für die α-Kette eines humanen T-Zellrezeptors, ein funktionelles Derivat oder ein Fragment davon kodiert und eine CDR3-Region, gebildet aus der Kombination eines Vα20-Gensegments mit einem Jα22-Gensegment umfaßt.

Die Länge des von dieser CDR3-Region kodierten Aminosäureabschnitts ist 11-14 Aminosäuren, vorzugsweise 12 oder 13 Aminosäuren. Besonders bevorzugt kodiert die CDR3-Region für eine der in den Sequenzprotokollen SEQ ID No. 2, 6 und 8 angegebene Aminosäuresequenz, eine damit mindestens 80 % und insbesondere mindestens 90 % identische Sequenz oder eine Sequenz, die für eine Aminosäuresequenz mit einer äquivalenten Erkennungsspezifität für die Peptidkomponente des T-Zellrezeptorliganden kodiert.

Ein weiterer Aspekt der vorliegenden Erfindung ist eine Nucleinsäure, die für die α-Kette eines humanen T-Zellrezeptors, ein funktionelles Derivat oder ein Fragment davon kodiert, und eine CDR3-Region umfaßt, ausgewählt aus:

(a) einer für die Aminosäuresequenz
$$Y\ C\ L\ (X_1 ... X_n)\ S\ A\ R\ Q\ L\ T\ F \qquad (I)$$
kodierenden Nucleotidsequenz,
wobei $X_1 ... X_n$ eine Sequenz von 3-5 Aminosäuren darstellt,
(b) einer Nucleotidsequenz, die für eine Aminosäuresequenz mit einer Identität von mindestens 80 % und insbesondere mindestens 90 % zur Aminosäuresequenz aus (a) kodiert, oder
(c) einer Nucleotidsequenz, die für eine Aminosäuresequenz mit einer äquivalenten Erkennungsspezifität für die Peptidkomponente des T-Zellrezeptor-Liganden kodiert.

Vorzugsweise ist die Aminosäuresequenz $X_1 ... X_n$ ausgewählt aus der Gruppe, bestehend aus den Aminosäuresequenzen VGG, VLSG, ATG, VSG, DSG, VVSG, ALAG, APSG und VGR. Besonders bevorzugt wird die Aminosäuresequenz $X_1 ... X_n$ ausgewählt aus den Aminosäuresequenzen VGG, VLSG und ATG.

Auffällig ist bei den erfindungsgemäßen tumorspezifischen CDR3α-Regionen insbesondere eine Länge von 12-13 Aminosäuren sowie ein gemeinsames Sequenzmotiv. So ist bei einer Länge der Sequenz $X_1 ... X_n$ von 3 Aminosäuren vorzugsweise $X_1 = V$ oder $A$, $X_2 = T$, $G$ oder $S$ und $X_3 = G$. Bei einer Länge der Sequenz $X_1 ... X_n$ von 4 Aminosäuren ist vorzugsweise $X_1 = V$ oder $A$, mindestens einer von $X_2$ oder $X_3$ $T$ oder $S$ und $X_4 = G$.

Eine Sequenzierung der β-Ketten aus beiden untersuchten Patienten ergab eine Kombination der Genseqmente Vβ22, Dβ1 bzw. Dβ2 und Jβ2.7 für den ersten Patienten und eine Kombination der Genseqmente von Vβ22, Dβ1 bzw. Dβ2 und Jβ2.1, Jβ2.3 bzw. Jβ2.7 für den zweiten Patienten.

Ein weiterer Aspekt der vorliegenden Erfindung ist somit eine Nucleinsäure, die für die β-Kette eines humanen T-Zellrezeptors, ein funktionelles Derivat oder ein Fragment davon kodiert und eine CDR3-Region gebildet aus der Kombination eines Vβ22- Gensegments eines Dβ1- oder Dβ2-Gensegments und eines Jβ-Gensegments, insbesondere eines Jβ2.1-, Jβ2.3 oder Jβ2.7-Gensegments umfaßt.

Die Länge des von dieser CDR3β-Region kodierten Aminosäureabschnitts ist 12-14 Aminosäuren, vorzugsweise 13 Aminosäuren. Weiterhin enthält diese CDR3β-Region bevorzugt ein gemeinsames Sequenzmotiv, nämlich X-T oder S-X-S, wobei X für eine beliebige Aminosäure steht und T oder S besonders bevorzugt T bedeutet. Insgesamt 70 % der untersuchten T-Zellrezeptoren weisen ein derartiges Sequenzmuster auf.

Noch ein weiterer Aspekt der vorliegenden Erfindung ist eine Nucleinsäure, die für die β-Kette eines humanen T-Zellrezeptors, ein funktionelles Derivat oder ein Fragment davon kodiert und eine CDR3-Region umfaßt, ausgewählt ist:

(a) einer für die Aminosäuresequenz

$\quad$ C A $(X'_1 ... X'_n)$ Y/D E Q Y F $\qquad$ (II)

kodierenden Nucleotidsequenz,

wobei $X'_1 ... X'_n$ eine Sequenz von 5-7 Aminosäuren darstellt,

(b) einer für die Aminosäuresequenz

$\quad$ C A $(X''_1 ... X''_n)$ N E Q F F $\qquad$ (III)

kodierenden Nucleotidsequenz,

wobei $X''_1 ... X''_n$ eine Sequenz von 5-7 Aminosäuren darstellt,

(c) einer für die Aminosäuresequenz

$\quad$ C A $(X'''_1 ... X'''_n)$ D T Q Y F $\qquad$ (IV)

kodierenden Nucleotidsequenz,

wobei $X'''_1 ... X'''_n$ eine Sequenz von 5-7 Aminosäuren darstellt,

(d) einer Nucleotidsequenz, die für eine Aminosäuresequenz mit einer Identität von minestens 80 % und insbesondere von mindestens 90 % zu einer Aminosäuresequenz aus (a), (b) oder/und (c) kodiert, oder

(e) einer Nucleotidsequenz, die für eine Aminosäuresequenz mit einer äquivalenten Erkennungsspezifität für die Peptidkomponente des T-Zellrezeptorliganden kodiert.

Die Aminosäuresequenz $X'_1 ... X'_n$ ist vorzugsweise aus der Gruppe ausgewählt, bestehend aus SSETNS, SSETSS, TSGTAS, RSGTGS, SSGTDS, SSGTRS, SSGSDS, SSSTGS, SSSTVS, SSSTLS, SSSTLF, SSSTAS, SSHTDS, SSDTLS UND SRWDSE. Besonders bevorzugt stellt die Aminosäuresequenz $X'_1 ... X'_n$ SSETNS, SSGTDS, TSGTAS oder RSGTGS dar. Die Aminosäuresequenz $X''_1 ... X''_n$ bedeutet vorzugsweise SSGTSSY oder SSDQGM. Die Aminosäuresequenz $X'''_1 ... X'''_n$ bedeutet vorzugsweise SADSFK.

Unter dem Begriff "funktionelles Derivat einer Kette eines humanen T-Zellrezeptors" im Sinne der vorliegenden Erfindung ist ein Polypeptid zu verstehen, das mindestens eine CDR3α- oder/und CDR3β-Region wie vorstehend definiert umfaßt und zusammen mit der jeweiligen komplementären Kette des humanen T-Zellrezeptors (oder einem Derivat einer solchen Kette) ein T-Zellrezeptor-Derivat bilden kann, das eine äquivalente Erkennungsspezifität für einen von einem MHC-Molekül präsentierten Peptidliganden wie der nicht-derivatisierte T-Zellrezeptor besitzt. Vorzugsweise weist ein derartiges T-Zellrezeptor-Derivat eine Bindungskonstante von mindestens $10^{-4}$ l/mol, vorzugsweise $10^{-4}$ bis $10^{-5}$ l/mol für den präsentierten Peptidliganden auf.

Die Herstellung funktioneller Derivate von Ketten eines humanen T-Zellrezeptors kann beispielsweise durch Deletion, Substitution oder/und Insertion von Abschnitten des für das jeweilige Polypeptid kodierenden Gens durch rekombinante DNA-Techniken erfolgen. Die Herstellung von rekombinanten T-Zellrezeptorketten ist beispielsweise bei Blank et al. (1993), Eur. J. Immunol. 23, 3057-3065; Lin et al. (1990) Sience 249: 677, Gregoire et al. (1991), Proc. Natl. Acad. Sci. USA, 88: 8077; Kappes und Tonegawa (1991), Proc. Natl. Acad. Sci. USA 88: 10619 und Ward (1991), Scand. J. Immunol. 34: 215, beschrieben. Auf diese Literaturstellen wird hiermit ausdrücklich Bezug genommen.

Besonders bevorzugte funktionelle Derivate von T-Zellrezeptorketten oder T-Zellrezeptoren sind Einzelketten-T-Zellrezeptoren, die beispielsweise aus den variablen Domänen der α- und β-Kette und einer konstanten Domäne zusammengesetzt sein können. Die Herstellung solcher Konstrukte ist bei Chung et al. (1994), Proc. Natl. Acad. Sci. USA 91: 12654-12658, beschrieben. Ein weiteres bevorzugtes Beispiel für funktionelle Derivate sind lösliche TCR-Fragmente, die als getrennte Polypeptide oder als Einzelketten-Polypeptide hergestellt werden können, vgl. z.B. Hilyard et al. (1994), Proc. Natl. Acad. Sci. USA 91: 9057-9061. Auch auf die Offenbarung in diesen Literaturstellen wird ausdrücklich Bezug genommen.

Ein weiterer Gegenstand der vorliegenden Erfindung ist ein Vektor, der mindestens eine Kopie einer erfindungsgemäßen Nucleinsäure enthält. Dieser Vektor kann ein prokaryontischer Vektor oder ein eukaryontischer Vektor sein. Beispiele für prokaryontische Vektoren sind Plasmide, Cosmide und Bakteriophagen. Derartige Vektoren sind bei Sambrook et al., Molecular Cloning. A Laboratory Manual, 2nd Edition (1989), Cold Spring Harbor Laboratory Press, in den Kapiteln 1-4 ausführlich beschrieben. Vorzugsweise ist der prokaryontische Vektor ein Plasmid.

Andererseits kann der Vektor auch ein eukaryontischer Vektor sein, z.B. ein Hefevektor, ein Pflanzenvektor (Bacolovirus) oder ein Säugervektor (ein Plasmidvektor oder ein viraler Vektor). Beispiele für eukaryontische Vektoren sind bei Sambrook et al, Supra, Kapitel 16 und Winnacker, Gene und Klone, Eine Einführung in die Gentechnologie (1985), VCH Verlagsgesellschaft, insbesondere in den Kapiteln 5, 8 und 10, beschrieben.

Noch ein weiterer Gegenstand der vorliegenden Erfindung ist eine Zelle, die eine erfindungsgemäße Nucleinsäure exprimiert oder eine Zelle, die mit einer erfindungsgemäßen Nucleinsäure oder mit einem erfindungsgemäßen Vektor transformiert ist. Die Zelle kann eine prokaryontische Zelle (z.B. eine gram-negative Bakterienzelle, insbesondere E.coli) oder eine eukaryontische Zelle (z.B. eine Hefe-, Pflanzen- oder Säugerzelle) sein. Beispiele für geeignete Zellen und Verfahren zum Einführen der erfindungsgemäßen Nucleinsäure in derartige Zellen finden sich in den obigen Literaturstellen.

Ein weiterer Gegenstand der vorliegenden Erfindung ist ein Polypeptid, das von einer erfindungsgemäßen Nucleinsäure kodiert ist. Besonders bevorzugt enthält das Polypeptid die variable Domäne der $\alpha$- oder/und $\beta$-Kette eines humanen T-Zellrezeptors.

Besonders bevorzugt ist ein Polypeptid, das T-Zellrezeptor-Eigenschaften aufweist und aus einer TCR-$\alpha$-Kette oder einem funktionellen Derivat davon sowie einer TCR-$\beta$-Kette oder einem funktionellen Derivat davon als Untereinheiten aufgebaut ist. Das Polypeptid kann aus zwei separaten Ketten zusammengesetzt sein oder als Einzelketten-Polypeptid vorliegen. Außerdem kann das Polypeptid auch in oligomerisierter Form vorliegen, wobei mindestens 2 und vorzugsweise 2-10 TCR$\alpha$- und TCR$\beta$-Ketten miteinander verknüpft vorliegen. Die Verknüpfung kann z.B. mittels bifuktioneller chemischer Linker erfolgen.

Noch ein weiterer Gegenstand der vorliegenden Erfindung ist ein Antikörper gegen ein erfindungsgemäßes Polypeptid, der gegen eine für die Erkennung des Peptidliganden verantwortliche Region des Polypeptids gerichtet ist. Dieser Antikörper kann ein polyklonales Antiserum, ein monoklonaler Antikörper oder ein Fragment eines polyklonalen oder monoklonalen Antikörpers (z.B. ein Fab-, F(ab)$_2$-, Fab'- oder F(ab')$_2$-Fragment) sein. Vorzugsweise ist der Antikörper gegen eine CDR3-Region des Polypeptids oder einen Bereich davon gerichtet. Derartige Antikörper können nach an sich bekannten Methoden durch Immunisierung eines Versuchstiers mit einem Peptid oder Polypeptid, welches eine erfindungsgemäße CDR3-Region enthält, und Gewinnung der resultierenden Antikörper aus dem Versuchstier erhalten werden. Monoklonale Antikörper können durch Fusion einer Antikörper-produzierenden B-Zelle des Versuchstiers mit einer Leukämiezelle nach der Methode von Köhler und Milstein oder eine Weiterentwicklung davon erhalten werden. Spezifische Beispiele für die Herstellung solcher Antikörper finden sich bei Choi et al. (1991), Proc. Natl. Acad. Sci. USA 88: 8357-8361 und Zumla et al. (1992), Hum. Immunol. 35: 141.

Noch ein weiterer Gegenstand der vorliegenden Erfindung ist eine T-Zelle, die einen erfindungsgemäßen T-Zellrezeptor enthält. Derartige T-Zellen können aus Patienten mit Nierenzellkarzinom isoliert und dann in vitro expandiert werden. Hierzu können beispielsweise die peripheren mononucleären Blutzellen eines Patienten durch Stimulation mit geeigneten Antigenen und anschließender Restimulation z.B. durch eine bestrahlte autologe Lymphoblastoid-Zellinie, Tumorzellen, Lymphoblastoidzellen plus Antigen oder autologe periphere Blutlymphozyten plus Antigen, erzeugt werden. Weitere Verfahren zur Gewinnung erfindungsgemäßer T-Zellen sind weiter unten beschrieben.

Die Erfindung betrifft auch eine pharmazeutische Zusammensetzung, die eine Nucleinsäure, ein Polypeptid, einen an das Polypeptid bindefähigen Peptidliganden, gegebenenfalls in Assoziation mit einem entsprechenden MHC-Molekül, einen Antikörper oder eine Zelle wie zuvor angegeben, als aktive Komponente, gegebenenfalls zusammen mit anderen aktiven Komponenten sowie pharmazeutisch üblichen Hilfs-, Zusatz-, oder Trägerstoffen enthält. Beispiele für weitere aktive Komponenten sind akzessorische stimulierende Komponenten, z.B. Cytokine, wie IL-2 und IL-4.

Die pharmazeutische Zusammensetzung kann zur Herstellung eines diagnostischen oder therapeutischen Mittels eingesetzt werden. Beispiele für diagnostische Anwendungen sind die Diagnose von Tumorerkrankungen oder einer Prädisposition für Tumorerkrankungen. Eine weitere bevorzugte diagnostische Anwendung ist die Überwachung des Krankheitsverlaufs bei einer Tumorerkrankung, z.B. nach einer Chemotherapie oder einem chirurgischen Eingriff.

Der Einsatz der pharmazeutischen Zusammensetzung als diagnostisches Mittel umfaßt vorzugsweise den Nachweis einer T-Zellsubpopulation, welche ein erfindungsgemäßes Polypeptid als T-Zellrezeptor exprimiert. Der Nachweis dieses T-Zellrezeptors kann beispielsweise auf Nucleinsäureebene, z.B. durch einen Nucleinsäure-Hybridisierungsassay, gegebenenfalls mit vorgeschalteter Amplifikation erfolgen. Andererseits kann der Nachweis auch auf Proteinebene durch einen Immunoassay unter Verwendung von spezifisch mit dem T-Zellrezeptor reagierenden Antikörpern erfolgen. Außerdem ist der Nachweis der T-Zellen beispielsweise auch über einen Test auf Bindung an spezifische Peptidliganden oder in einem Aktivitätstest, bei dem die spezifische cytotoxische Wirkung der T-Zellen oder die Freisetzung von Cytokinen wie TNF oder IFN$\gamma$ bestimmt wird, möglich.

Weiterhin kann die erfindungsgemäße pharmazeutische Zusammensetzung auch auf therapeutischem Gebiet angewandt werden, insbesondere zur Prävention oder Therapie einer Tumorerkrankung, z.B. eines Nierenzellkarzinoms. Diese therapeutische Anwendung kann beispielsweise darauf beruhen, daß eine Stimulation des Wachstums von T-Zellen, die den tumorspezifischen T-Zellenrezeptor exprimieren, in vitro oder in vivo erfolgt. Die Wachstumsstimulierung in vivo kann beispielsweise durch Verabreichung des Peptidliganden des T-Zellrezeptors oder/und des gesamten Moleküls, aus dem der Peptidligand stammt, oder eines Fragments davon erfolgen. Weiterhin kann die Wachstumsstimulierung in vivo auch durch Verabreichung eines spezifisch den T-Zellrezeptor durch Bindung aktivierenden Antikörpers, z.B. eines monoklonalen Antikörpers oder eines monoklonalen Antikörperfragments bewirkt werden.

Andererseits kann die Wachstumsstimulierung der T-Zellen auch in vitro durchgeführt werden, beispielsweise durch Gewinnung spezifischer T-Zellen aus dem Patienten, in vitro-Expansion und anschließende Verabreichung der expandierten T-Zellen als Tumorvakzine. Die Gewinnung von T-Zellen aus einem Patienten, die einen tumorspezifischen T-Zellrezeptor exprimieren, erfolgt vorzugsweise derart, daß man eine T-Zellen enthaltende Probe aus dem Patienten, z.B. eine Blutprobe und vorzugsweise eine aus dem Tumorgewebe stammende Probe mit einem spezifisch an die CDR3-Region des T-Zellrezeptors bindenden Mittel in Kontakt bringt, die mit dem Mittel reagierenden T-Zellen identifiziert und gegebenenfalls von anderen T-Zellen abtrennt. Das an die CDR3-Region des T-Zellrezeptors bindende Mittel wird vorzugsweise ausgewählt aus dem Peptidliganden der T-Zellen, einem Peptidligand-MHC-Komplex oder/und einem Anti-TCR-Antikörper. Gegebenenfalls kann die in vitro-Expansion zusätzlich in Gegenwart costimulatorischer Faktoren durchgeführt werden, z.B. Anti-CD28-Antikörpern. Vorzugsweise wird zur Erleichterung der Abtrennung der gewünschten T-Zellsubpopulation das Mittel in einer immobilisierten oder immobilisierbaren Form verwendet.

Die Gewinnung von T-Zellen, die einen tumorspezifischen T-Zellrezeptor exprimiert, kann jedoch auch auf andere Weise erfolgen, z.B. durch Einführen von Nucleinsäuresequenzen, die für den T-Zellrezeptor kodieren, in eine T-Zellinie, vorzugsweise eine cytotoxische T-Zellinie. In dieser transfizierten T-Zellinie erfolgt dann eine Expression des T-Zellrezeptors. Auf diese Weise sind T-Zellen, welche einen tumorspezifischen T-Zellrezeptor exprimieren, in größeren Mengen erhältlich.

Noch eine andere Möglichkeit zur Gewinnung von T-Zellen, die einen tumorspezifischen T-Zellrezeptor exprimieren, besteht darin, daß man Nucleinsäuresequenzen, die für den T-Zellrezeptor kodieren, in die Keimbahn eines Tieres einführt und die T-Zellen aus dem resultierenden transgenen Tier oder Nachkommen davon gewinnt. Vorzugsweise werden transgene Mäuse hergestellt. Weiterhin ist bevorzugt, daß die transgenen Mäuse neben dem T-Zellrezeptor auch das humane CD8-Molekül oder/und dar humane HLA-A*0201-Molekül exprimieren.

Ein weiterer Gegenstand der vorliegenden Erfindung ist somit auch ein transgenes Tier, welche T-Zellen besitzt, die einen tumorspezifischen T-Zellrezeptor exprimieren. Vorzugsweise ist dieses transgene Tier ein Nagetier, insbesondere eine Maus.

Schließlich betrifft die Erfindung auch ein Verfahren zur Identifizierung von Peptidliganden eines erfindungsgemäßen T-Zellrezeptors. Dieses Verfahren umfaßt vorzugsweise die Schritte:

(a) Gewinnen von RNA aus Tumorgewebe,
(b) Überführen der RNA in doppelsträngige cDNA-Moleküle,
(c) Einbringen der cDNA-Moleküle in Wirtszellen, wobei eine cDNA-Bank erhalten wird,
(d) Transfizieren von eukaryontischen Empfängerzellen mit Aliquots der cDNA-Bank, wobei (i) eine Cotransfektion mit HLA-A*0201-DNA erfolgt oder (ii) HLA-A*0201 positive Empfängerzellen verwendet werden,
(e) Testen der transfizierten Empfängerzellen auf Fähigkeit zur Stimulation von T-Zellen, beispielsweise zur Proliferation oder zur Sekretion von Cytokinen wie etwa TNF, wobei z.B. die Lyse von TNF-sensitiven Zellen untersucht werden kann,
(f) Identifizieren einer cDNA-Sequenz, die für das Antigen, welches den Peptidliganden enthält, kodiert und
(g) Identifizieren der Sequenz des Peptidliganden.

Weiterhin wird die Erfindung durch nachfolgende Beispiele, Abbildungen und Sequenzprotokolle erläutert. Es zeigen

| | |
|---|---|
| SEQ ID No. 1: | Die Nucleotidsequenz der TCR-$\alpha$-Kette eines erfindungsgemäßen T-Zellrezeptors, wobei bp 55-324/325 für das TCR-V$\alpha$20-Gensegment kodieren, bp 325/326 für das TCR J$\alpha$22-Gensegment kodieren, bp 381-804 für das TCR-C$\alpha$-Gensegment kodieren und bp 805-1341 einen 3'-untranslatierten Bereich darstellen, |
| SEQ ID No. 2: | Die Aminosäuresequenz zu der in SEQ ID. No. 1 dargestellten Nucleotidsequenz, |
| SEQ ID No. 3: | Die Nucleotidsequenz der TCR-$\beta$-Kette eines erfindungsgemäßen T-Zellrezeptors, wobei bp 1-63 für das Leaderpeptid kodieren, bp 64-341 für das TCR-V$\beta$22-Gensegment kodieren, bp 342-345 N-Nucleotide sind, bp 346-349 für das TCR-D$\beta$2 Gensegment kodieren, bp 350 ein N-Nucleotid ist, bp 351-398 für das TCR-J$\beta$2.7-Gensegmentkodieren und bp 399-936 für das TCR-C$\beta$-Gensegment kodieren, |
| SEQ ID No. 4: | Die Aminosäuresequenz zu der in SEQ ID No. 3 angegebenen Nucleotidsequenz, |

SEQ ID No. 5 und 6:    Nucleotid- und Aminosäuresequenzen der CDR3α-Region eines erfindungsgemäßen T-Zellrezeptors,

SEQ ID No. 7 und 8:    Nucleotid- und Aminosäuresequenzen der CDR3α-Region eines erfindungsgemäßen T-Zellrezeptors,

SEQ ID No. 9 und 10:    Nucleotid- und Aminosäuresequenzen der CDR3β-Region eines erfindungsgemäßen T-Zellrezeptors,

SEQ ID No. 11 und 12:    Nucleotid- und Aminosäuresequenzen der CDR3β-Region eines erfindungsgemäßen T-Zellrezeptors,

SEQ ID No. 13 und 14:    Nucleotid- und Aminosäuresequenzen der CDR3β-Region eines erfindungsgemäßen T-Zellrezeptors,

SEQ ID No. 15 und 16:    Nucleotid- und Aminosäuresequenzen der CDR3β-Region eines erfindungsgemäßen T-Zellrezeptors,

SEQ ID No. 17 und 18:    Nucleotid- und Aminosäuresequenzen der CDR3β-Region eines erfindungsgemäßen T-Zellrezeptors,

SEQ ID No. 19 und 20:    Nucleotid- und Aminosäuresequenzen der CDR3β-Region eines erfindungsgemäßen T-Zellrezeptors,

SEQ ID No. 21    die Nucleotidsequenz des TCRα-spezifischen Primers P-CαST,

SEQ ID No. 22    die Nucleotidsequenz des TCRβ-spezifischen Primers P-CβST,

Abb. 1    Nucleotid- und Aminosäuresequenzen von CDR3α-Regionen aus tumorspezifischen TCR, die durch in situ-Sequenzierung von T-Zellen des Patienten 26 bestimmt wurden,

Abb. 2    Nucleotid- und Aminosäuresequenzen von CDR3β-Regionen aus tumorspezifischen TCR, die durch in situ-Sequenzierung von T-Zellen des Patienten 26 bestimmt wurden,

Abb. 3    Nucleotid- und Aminosäuresequenzen von CDR3α-Regionen aus tumorspezifischen TCR, die durch in situ-Sequenzierung von T-Zellen des Patienten 22 bestimmt wurden und

Abb. 4    Nucleotid- und Aminosäuresequenzen von CDR3β-Regionen aus tumorspezifischen TCR, die durch in situ-Sequenzierung von T-Zellen des Patienten 22 bestimmt wurden.

Beispiel 1

Analyse von T-Zellrezeptoren in HLA-A2-Patienten mit Nierenzellkarzinom

Im Nierenzellpatienten 26 wurden cytotoxische CD8[+]-T-Zellen identifiziert, die autologe Tumorzellen in einem HLA-A2-restringierten Mechanismus lysieren. Die T-Zellen besitzen eine hohe Tumorspezifität, da Kurzzeitkulturen von normalen Nierenzellen nicht erkannt werden. Die von den TIL des Patienten 26 erkannte Determinante wurde auch auf anderen Tumoren von Patienten gefunden, welche das HLA-A2-Gen, insbesondere das weit verbreitete HLA-A*0201-Allel, tragen. Normale Nierenzellen dieser Patienten wurden nicht lysiert. Diese Ergebnisse zeigen, daß die Nierenkarzinomzellen des Patienten 26 eine Tumordeterminante exprimieren, d.h. einen Tumor-assoziierten Peptid/HLA-A2-Komplex, der auch auf Tumoren anderer Patienten vorliegt.

Zur Identifizierung und Sequenzierung von tumorspezifischen TCR wird aus T-Zellen Gesamt-RNA isoliert. Hierzu werden die Zellen in Suspension mit PBS gewaschen und das Zellpellet mit 0,2 ml RNazol-B pro $1 \times 10^6$ Zellen resuspendiert. Für die RNA-Extraktion aus Gewebe werden 2 ml RNazol-B pro 100 mg Gewebe eingesetzt. Nach mehrfachem mechanischen Resuspendieren der Lysate und gegebenenfalls Zugabe von Hefe tRNA als Trägermatrix erfolgt die RNA-Extraktion durch Zugabe von 0,2 ml Chloroform pro 2 ml Homogenisat, nachfolgendem Mischen für 15 sek. und 5-minütiger Lagerung auf Eis.

Nach einem Zentrifugationsschritt von 12000 g für 15 min. bei 4 °C wird die wässrige Phase abgenommen und in ein neues Reaktionsgefäß überführt. Die erste Präzipitation der RNA erfolgt durch Zugabe eines identischen Volumens Isopropanol und anschließender Lagerung für mindestens 15 min. bei 4 °C. Nach Zentrifugation für 15 min. bei 12000 g und 4 °C wird die RNA als weißes Pellet am Grund des Gefäßes erhalten.

Nach Verwerfen des Überstandes wird das RNA-Pellet durch kurzes Mischen in 75 % Ethanol von Salzen gereinigt. Nach Zentrifugation (7500 g, 4 °C, 8 min.) wird das Pellet in 175 μl mit Diethylpyrocarbonat (DEPC) behandeltem Wasser gelöst und mit 500 μl Ethanol und 75 μl 2 M Nacl für mindestens 1 h bei - 20 °C erneut präzipitiert. Die Zentrifugations- und Waschschritte nach der zweiten Präzipitation erfolgen wie bei der ersten Fällung beschrieben. Nach Lufttrocknung des Pellets wird die RNA in $H_2O$-DEPC oder 0,5 % SDS, pH 6,5 bis 7,0 oder 1 mM EDTA, pH 7,0 resuspendiert.

Anschließend wird aus der RNA durch reverse Transkription cDNA synthetisiert. Hierzu werden 3 μg Gesamt-RNA mit 30 ng P-CαST (ein für die TCR-α-Kette spezifischer Primer mit der in SEQ ID No. 21 gezeigten Sequenz 5'-CAC TGA AGA TCC ATC ATC TG-3') und 30 ng P-CβST (ein für die TCR-β-Kette spezifischer Primer mit der in SEQ ID No. 22 gezeigten Sequenz 5'-TAG AGG ATG GTG GCA GAC AG-3') in einem Reaktionsvolumen von 10 μl für 10 min bei 55 °C inkubiert. Anschließend werden 38 µl RAV-2-RT-Puffer (100 mM Tris-HCl pH 8,3; 140 mM KCl; 10 mM MgCl$_2$; 2 mM Dithiothreitol, jeweils 0,1 mM dNTP), 1 µl (0,75 U) rRNasin und 1 µl (18 U) reverse Transkriptase zupipettiert. Die reverse Transkription erfolgt für 1 h bei 42 °C, gefolgt von einem Denaturierungsschritt bei 68 °C für 5 min. Die Lagerung bis zum Verbrauch erfolgt bei - 20 °C.

Anschließend wird eine Polymerase-Kettenreaktion durchgeführt. Die Primer können biotinyliert sein, um eine nachfolgende Aufreinigung der PCR-Produkte durch Kopplung an eine magnetische partikuläre Festphase (Streptavidin-beschichtete Beads) zu ermöglichen.

Die PCR wird unter Verwendung einer thermostabilen DNA-Polymerase mit folgendem Reaktionsschema durchgeführt:

95 °C 5 min. Prädenaturierung (nur am Anfang)
95 °C 30 sek. DNA-Denaturierung
56 °C 1 min. Annealing
72 °C 1 min. Extension
72 °C 10 min. Auffüllen aller Einzelstränge in der Reaktionslösung (nur am Ende).

Die Anzahl von Reaktionszyklen bei der PCR ist in der Regel 30.

Die auf diese Weise erhaltenen PCR-Fragmente werden sequenziert.

Bei Kultivierung und periodischer Restimulierung der cytotoxischen T-Zellen aus dem Patienten 26 über eine Dauer von 62 bzw. 74 Tagen wird ein einheitlicher CD8$^+$ T-Zellklon erhalten. Die Nucleotid- und Aminosäuresequenz der TCR-α-Kette dieses T-Zellklons aus dem Patienten 26 sind in SEQ ID No. 1 und 2 angegeben. Die Nucleotid- und Aminosäuresequenz der TCR-β-Kette sind in SEQ ID No. 3 und 4 angegeben.

Bei einer nur 24-tägigen Kultivierung der tumorinfiltrierenden Lymphozyten aus dem Patienten 26 wurde kein einheitlicher T-Zellklon, sondern ein Gemisch aus mehreren T-Zellspezies gefunden. Die CDRα-Regionen dieser T-Zellspezies enthielten neben der in SEQ ID No. 2 gezeigten Aminosäuresequenz insgesamt 2 weitere Sequenzen (SEQ ID No. 5 und 6 bzw. 7 und 8) Die CDR3β-Regionen enthielten neben der in SEQ ID No. 4 gezeigten Aminosäuresequenz weitere nah verwandte Sequenzen (SEQ ID No. 9 und 10, 11 und 12, 13 und 14, 15 und 16, 17 und 18 bzw. 19 und 20).

Weiterhin wurde eine in situ-Sequenzierung von T-Zellen des Patienten 26, d.h. eine Sequenzierung ohne vorherige Kultivierung durchgeführt. Dabei wurde für die CDR3α-Region eine Reihe von Einzelsequenzen erhalten, die in Abb. 1 angegeben ist. Circa 60 % aller Sequenzen der α-Kette entsprechen den bereits zuvor angegebenen Sequenzen. Weitere 20 % entsprechen sehr ähnlichen Sequenzen.

Auch für die CDR3-Regionen der β-Kette wurde festgestellt, daß insgesamt 70 % der untersuchten T-Zellen des Patienten 26 ein sehr ähnliches Sequenzmuster aufwiesen (Abb. 2).

Eine Analyse von peripheren Blutproben aus dem Patienten 26 auf T-Zellrezeptoren, welche die Merkmale der tumorspezifischen T-Zellrezeptoren aufweisen, wurde über insgesamt 4 Jahre durchgeführt. Dabei wurde festgestellt, daß solche Sequenzen mit einer Häufigkeit von nur etwa 1/150 000 T-Zellen vorkommen.

Durch Cytotoxizitätsuntersuchungen wurde festgestellt, daß die aus dem Patienten 26 gewonnenen tumorspezifischen T-Zellen auch Tumorzellen des ebenfalls das HLA-A*0201-Allel tragenden Patienten 22 lysieren konnten. Tumorinfiltrierende T-Zellen aus dem Patienten 22 konnten wiederum Tumorzellen aus dem Patienten 26 lysieren. Eine Sequenzierung der T-Zellrezeptoren aus dem Patienten 22 ergab die für die CDR3α-Region die in Abb. 3 und für die CDR3β-Region die in Abb. 4 dargestellten Ergebnisse.

Beispiel 2

Expression von T-Zellrezeptoren

2.1 Expression tumorspezifischer T-Zellrezeptoren in humanen oder murinen T-Zellinien

Die in Beispiel 1 identifizierten Nucleinsäuresequenzen, die für tumorspezifische TCR-α- und β-Ketten kodieren, werden in eukaryontische humane und murine Expressionsvektoren kloniert. Der humane Expressionsvektor ist bei Chung et al. (Proc. Natl. Acad. Sci. USA 92 (1995): 3712-3716) beschrieben. Die murinen Vektoren sind bei Gabert et al. (Cell 50 (1987: 545-554) und Gregoire et al. (Proc. Natl. Acad. Sci. USA 88 (1991): 8077-8081) beschrieben.

Die Klonierung der TCR-DNA kann entweder aus rearrangierter genomischer oder aus cDNA durchgeführt werden. Prinzipiell stehen zwei Klonierungsstrategien zur Verfügung: Erstens die Isolation von sehr langen TCR-α- und β-DNA-Fragmenten aus dem Genom reifer T-Zellen, die mehrere Kb-lange 5'-flankierende Sequenzen mit allen zur Expression benötigten regulatorischen Elementen enthalten. Alternativ können Vektoren gewählt werden, die bereits die natürlichen 5'-regulatorischen Elemente enthalten, und in die nur kurze, für die variablen Regionen kodierende Fragmente einkloniert werden müssen (Kouskoff et al. J. Immunol. Methods 180 (1995): 273-280). Bei der letztgenannten Methode wird die Sequenz der variablen Region (einschließlich der Leadersequenz) nach Amplifikation mittels spezifischer PCR durch Sequenzierung auf Fehler hin untersucht und anschließend nach Verdauung mit den entsprechenden Restriktionsendonucleasen in den Vektor eingebracht.

Die PCR-$\alpha$- und $\beta$-Ketten können entweder in einen gemeinsamen oder in zwei verschiedene Vektoren kloniert werden. Jeder der verwendeten Vektoren enthält einen Selektionsmarker, der nach Transfektion der Empfängerzellen mit dem rekombinanten Plasmid die positive Selektion von erfolgreich transfizierten Zellen erlaubt. Bevorzugte Selektionsmarker sind z.B. das Gen für die Neomycin-Resistenz (Neo) oder das Gen für die Xanthin-Guanin-Phosphoribosyl-Transferase (GPT).

2.2 Expression funktioneller T-Zellrezeptoren als Einzelkettenkonstrukte

Analog zu Antikörpern können TCR als Einzelkettenkonstrukte in eukaryontischen Zellen zur Expression gebracht werden (Chung et al., Proc. Natl. Acad. Sci. USA 91 (1994): 12654-12658). Hierbei wird ein Konstrukt hergestellt, das neben den variablen Domänen der TCR-$\alpha$- und $\beta$-Kette ebenfalls die konstante Domäne der $\beta$-Kette enthält. Die einzelnen Domänen werden wie in Beispiel 1 beschrieben nach Isolation der entsprechenden RNA und reverser Transkription mittels PCR amplifiziert. Dabei werden an den Enden der Amplifikationsprodukte geeignete Restriktionsschnittstellen eingefügt. Die einzelnen Fragmente werden dann in einen eukaryontischen Expressionsvektor (z.B. pBJ-Neo), der einen positiven Selektionsmarker trägt, wie folgt aneinandergefügt: Die variablen TCR-$\alpha$- und $\beta$-Domänen, bestehend aus Leader-, V-(D-) und J-Exon werden durch eine Linkersequenz, z.B. ein für die Aminosäuresequenz $(GGGGS)_3$ kodierendes DNA-Fragment, getrennt. Das Exon für die konstante TCR-$\beta$-Domäne wird direkt an die variable $\beta$-Domäne ligiert. An das 3'-Ende dieses Konstrukts können alternativ kodierende Sequenzen für einen GPI-Anker (Lin et al., Science 249 (1990): 677-679) oder z.B. den Transmembranteil und die Intrazellulärdomäne der CD3$\zeta$ -Kette (Engel et al., Science 256 (1992): 1318-1321) ligiert werden. Nach Transfektion dieser Konstrukte in eukaryontische Zellen ermöglicht ersteres die Herstellung löslicher TCR-Moleküle, die als Immunogen zur Herstellung von Antikörpern verwendet werden können. Letzteres erlaubt die funktionelle Analyse des Konstrukts in biologischen Systemen.

2.3 Herstellung von löslichen humanen TCR-Fragmenten in E.coli

Größere Mengen löslicher TCR-Fragmente können in E.coli als Einzelketten-Polypeptide hergestellt werden (Hilyard et al., Proc. Natl. Acad. Sci. USA 91 (1994): 9057-9061).

Dazu werden verschiedene Gene bzw. Genfragmente in einen induzierbaren prokaryontischen Vektor, z.B. pUC19 kloniert. Die zu ligierenden Fragmente werden mittels spezifischer PCR reamplifiziert, wobei geeignete Restriktionsschnittstellen angefügt werden.

Folgende Fragmente werden in der aufgeführten Reihenfolge in den Vektor kloniert:

1. eine prokaryontische Signalsequenz, z.B. die pelB-Leadersequenz aus dem Pektat-Lyasegen von Erwinia carolovora (Ward et al., Nature 341 (1989): 8646-8650), die eine Sekretion des Polypeptids in das Periplasma des Wirtsbakteriums bewirkt.

2. Die variablen PCR-$\alpha$- und $\beta$-Kettenfragmente aus einem tumorspezifischen TCR. Diese Fragmente werden vorzugsweise durch einen Linker, z.B. den in Beispiel 2.2 angegebenen Linker getrennt, wodurch eine bessere Löslichkeit und höhere Flexibilität des synthetisierten Moleküls erreicht wird.

3. Eine für einen Schwanz aus mehreren, z.B. 6 Histidinresten kodierende Nucleotidsequenz, wodurch die Isolierung des rekombinanten Polypeptids durch Affinitäts-Chromatographie, z.B. durch Nickel-Chelat-Chromatographie ermöglicht wird.

Beispiel 3

Herstellung von Antikörpern gegen tumorspezifische T-Zellrezeptoren

Zur Herstellung von Antiseren bzw. monoklonalen Antikörpern gegen tumorspezifische TCR werden Mäuse mit dem entsprechenden Antigen immunisiert. Die Immunisierung erfolgt nach den bei Harlow, E. und David, C., Antibodies. A Laboratory Manual, Cold Spring Harbor Laboratory, 1988, angegebenen Protokollen. Als Antigene können beispielsweise TCR-exprimierende Zellen (Beispiel 2.1) oder lösliche TCR (Beispiel 2.2 oder Beispiel 2.3) gewählt werden.

Alternativ können die zur Immunisierung verwendeten löslichen TCR auch als chimäre Proteine hergestellt werden, die aus einer variablen TCR-Region, einer verkürzten konstanten TCR-Region und aus einer konstanten Immunglobinregion bestehen (vgl. z.B. Gregoire et al. (1991), Supra). Dazu werden die spezifischen variablen TCR-$\alpha$- und $\beta$-Regionen in jeweils ein Plasmid kloniert, das bereits das erste Exon die entsprechende C-Region und eine IgGk-Domäne enthält. Beide Plasmide enthalten zusätzlich einen positiven Selektionsmarker und die zur korrekten Expression benötigten regulatorischen Elemente. Beide Plasmide werden dann zur Transfektion einer Mausmyelomzellinie verwendet, die keine endogenen schweren und leichten Ig-Ketten exprimiert. Nach erfolgreicher Transfektion werden beiden chimären Ketten synthetisiert und präferentiell als Heterodimer sezerniert.

Alternativ kann ein TCR-Protein-Antigen zur Immunisierung von Mäusen auch wie folgt konstruiert werden: An ein TCR-Gensegment, bestehend aus (D-), J- und C-Gensegmenten aus einem Maus-T-Zellhybridom wird ein humanes V-Gensegment fusioniert, d.h. die Gensegmente werden in dieser Reihenfolge in einen eukaryontischen Expressionsvektor kloniert (Choi et al., Proc. Natl. Acad. Sci. USA 88 (1991): 8357-8361). Die humane Sequenz wird durch Amplifikation der V-Region aus der entsprechenden cDNA mittels PCR gewonnen. Solche Konstrukte werden dann zur Transfektion von Maus-T-Zellhybridomen verwendet, die alle Bestandteile außer den entsprechend transfizierten Ketten zur Verfügung stellen. Da die Plasmide ebenfalls für Selektionsmarker kodieren, können Transfektanten durch das entsprechende Medium positiv selektioniert werden. Da diese Transfektanten Maus T-Zellen darstellen, die eine humane V-Region exprimieren, erfolgt bei Immunisierung von Mäusen mit solchen Zellen nur eine Antikörperbildung gegen diese "fremde" humane Sequenz.

Beispiel 4

Identifizierung der Peptidliganden von tumorspezifischen T-Zellen

Poly-A$^+$-mRNA wird aus einer Nierenzellkarzinomlinie unter Verwendung eines kommerziellen Kit (Fastrack/Invitrogen) isoliert und unter Verwendung des Superscript Choice System Kit (Gibco) unter Verwendung eines NotI/Oligo-dT-Primer für die Erststrangsynthese in Doppelstrang-cDNA überführt. Die cDNA wird mit BstXI-Adaptoren ligiert und mit NotI gespalten. Hochmolekulare, größenfraktionierte cDNA wird selektioniert und in den mit BstXI und NotI gespaltenen Vektor pcDNAI/Amp (Invitrogen) kloniert.

E.coli DH5-$\alpha$-Zellen werden mit den rekombinanten Plasmiden durch Elektroporation transformiert und mit Ampicillin selektioniert. Die auf diese Weise erhaltene c-DNA-Bank wird in 1500 Pools aus jeweils ungefähr 100 Klonen unterteilt. Jeder Pool wird bis zur Sättigung amplifiziert und die Plasmid-DNA daraus durch alkalische Lyse ohne Phenolextraktion gewonnen.

Ungefähr jeweils 100 ng Plasmid-DNA eines Pools werden zusammen mit 50 ng Plasmid-DNA des gleichen Vektors, welcher die HLA-A*0201-cDNA (Genbank, ACC-Nr.: M32322, K02883, M84379, X02457) trägt, in 15000 COS7-Zellen nach der DEAE-DextranChloroquin-Methode transfiziert. Alternativ können die COS7-Zellen auch mit der HLA-A*0201-DNA transfiziert und die auf diese Weise erhaltenen stabilen Transfektanten als Empfängerzellen verwendet werden.

24-48 Stunden nach der Transfektion werden die COS7-Zellen auf ihre Fähigkeit zur Stimulierung der Freisetzung von TNF durch tumorspezifische cytotoxische T-Zellen (CTL) getestet. Ein Test wird jeweils mit 200 Pools, d.h. 200 unabhängigen Transfektionen von COS7-Zellen durchgeführt.

Hierzu werden 3000 CTL in die COS7-Transfektanten enthaltenden Vertiefungen von Mikrotiterplatten gegeben. Nach 18 Stunden wird der Überstand des Mediums gesammelt und dessen TNF-Gehalt unter Verwendung eines Aktivitätstests bestimmt, bei dem TNF-sensitive Zellinien, wie etwa die Maus-Fibroblasten-Zellinien WEHI 164 oder L929 durch TNF lysiert werden. Lebensfähige Kulturen können von lysierten Zellen durch einen colorimetrischen Test unter Verwendung von 3-(4,5-Dimethylthiazol-2-yl)2,5-diphenyltetrazoliumbromid (MTT) unterschieden werden.

Für jede positive Mikrokultur wird eine neue Runde der COS7-Transfektion durchgeführt, wobei jeweils kleinere Pools von Bakterien aus dem Ursprungspool mit insgesamt 100 Klonen verwendet wurden. Diese Prozedur wird wiederholt, bis ein einziges Plasmid identifiziert wird, welches die TNF-Freisetzung aus den spezifischen TCL nach Coexpression mit der HLA-A*0201-cDNA in COS7-Zellen induzieren kann.

Die Sequenz der Plasmidinsertion wird durch Standardmethoden bestimmt. Die Bestätigung, daß diese Sequenz für das Tumorpeptid kodiert, erfolgt durch Transfektion normaler humaner HLA-A*0201-Zellen, die nicht durch die tumorspezifischen CTL lysiert werden. Diese Zellen werden nach Transfektion mit der entsprechenden cDNA für eine Lyse sensitiv. Weiterhin wird die tumorspezifische Expression der identifizierten cDNA durch Northern Blot unter Verwendung der cDNA als Sonde bestimmt. Diese Sonde wird zur Hybridisierung an mRNA aus verschiedenen Tumor-Zellinien normalen Gewebeproben verwendet.

Das tumorspezifische Peptid kann durch unterschiedliche Methoden identifiziert werden. Die korrespondierende Proteinsequenz wird aus der cDNA-Sequenz abgeleitet und nach Bindemotiven durchmustert, die in anderen an HLA-A*0201 bindenden Peptiden identifiziert worden sind. Synthetische Peptide, die mit potentiellen HLA-A*0201-Binderegionen überlappen, werden dann auf ihre Fähigkeit zur Aktivierung von CTL nach Inkubation mit HLA-A*0201-Zellen getestet. Alternativ können überlappende Peptide von 8-9 Aminosäuren Länge durch Synthese erzeugt und auf ähnliche Weise getestet werden.

Beispiel 5

Herstellung transgener Mäuse

Gesamt-RNA wird aus einem spezifischen T-Zellklon isoliert, und cDNA wird durch reverse Transkription synthetisiert (vgl. Beispiel 1). Unter Verwendung von für die V-Region spezifischen Primern wird TCR-cDNA für die V$\alpha$- und V$\beta$-Regionen amplifiziert und in TCR-Genkassetten kloniert, die konstante Regionen und die für die Expression notwendigen Regulationselemente enthalten. Es sind separate Kassetten für TCR-$\alpha$- und TCR-$\beta$-Sequenzen bekannt, die jeweils einen verschiedenen Selektionsmarker tragen (Kouskoff et al. (1995), Supra).

Fertilisierte Mausoocyten werden gleichzeitig mit DNA sowohl aus den TCR-$\alpha$- als auch TCR-$\beta$-Kassetten mikroinjiziert. Die injizierten Oocyten werden in weibliche Mäuse rückübertragen (Mellor, A.L., Transgenesis and the T cell receptor. in: T cell receptors (1995), J. I. Bell, M. J. Owen, und E. Simpson, eds, pp194-223, Oxford University Press, Oxford, New York, Tokyo).

Die Einführung produktiv umgelagerter TCR-Gene in die Maus hat einen großen Einfluß auf das TCR-Repertoir, da umgelagerte TCR-Fremdgene die weitere Umlagerung von endogenen TCR-Genen verhindern. Folglich exprimieren nahezu alle Thymocyten und T-Zellen den heterologen TCR-Klonotyp, so daß das TCR-Repertoir in solchen Mäusen im wesentlichen monoklonal ist.

Transgene Mäuse werden durch Genotypanalyse unter Verwendung von Sonden identifiziert, die spezifisch für die im Fremdgen enthaltene DNA sind, welche im Mausgenom nicht vorkommt. Dies kann entweder durch Southern Blot-Hybridisierung oder vorzugsweise durch PCR erfolgen.

Transgene Nachkommen der Mäuse werden durch Kreuzung mit nicht-transgenen Mäusen eines geeigneten Stammes, Typisierung der Nachkommenschaft und Verwendung zur Weiterkreuzung erhalten.

**Patentansprüche**

1. Nucleinsäure, die für die $\alpha$-Kette eines humanen T-Zellrezeptors, ein funktionelles Derivat oder ein Fragment davon kodiert und eine CDR3-Region gebildet aus der Kombination eines V$\alpha$20-und eines J$\alpha$22-Gensegments umfaßt.

2. Nucleinsäure, die für die $\alpha$-Kette eines humanen T-Zellrezeptors, ein funktionelles Derivat oder ein Fragment davon kodiert, und eine CDR3-Region umfaßt, ausgewählt aus:

   (a) einer für die Aminosäuresequenz
       $Y C L (X_1 ... X_n) S A R Q L T F$       (I)
   kodierenden Nucleotidsequenz,
   wobei $X_1 ... X_n$ eine Sequenz von 3-5 Aminosäuren darstellt,
   (b) einer Nucleotidsequenz, die für eine Aminosäuresequenz mit einer Identität von mindestens 80 % zur Aminosäuresequenz aus (a) kodiert, oder
   (c) einer Nucleotidsequenz, die für eine Aminosäuresequenz mit einer äquivalenten Erkennungsspezifität für die Peptidkomponente der T-Zellrezeptor-Liganden kodiert.

3. Nucleinsäure nach Anspruch 2,
   **dadurch gekennzeichnet**,
   daß die Aminosäuresequenz $X_1 \ldots X_n$ ausgewählt ist aus der Gruppe, bestehend aus den Aminosäuresequenzen VGG, VLSG, ATG, VSG, DSG, VVSG, ALAG, APSG und VGR.

4. Vektor,
   **dadurch gekennzeichnet**,
   daß er mindestens eine Kopie einer Nucleinsäure nach einem der Ansprüche 1 bis 3 enthält.

5. Zelle,
   **dadurch gekennzeichnet**,
   daß sie eine Nucleinsäure nach einem der Ansprüche 1 bis 3 exprimiert.

6. Polypeptid,
   **dadurch gekennzeichnet**,
   daß es von einer Nucleinsäure nach einem der Ansprüche 1 bis 3 kodiert ist.

7. Nucleinsäure, die für die β-Kette eines humanen T-Zellrezeptors, ein funktionelles Derivat oder ein Fragment davon kodiert und eine CDR3-Region gebildet aus der Kombination eines Vβ22-Gensegments, eines Dβ1- oder Dβ2-Genseqments und eines Jβ-Gensegments, insbesondere eines Jβ2.1-, Jβ2.3 oder Jβ2.7-Gensegments umfaßt.

8. Nucleinsäure, die für die β-Kette eines humanen T-Zellrezeptors, ein funktionelles Derivat oder ein Fragment davon kodiert und eine CDR3-Region umfaßt, ausgewählt aus:

   (a) einer für die Aminosäuresequenz
   $$C \, A \, (X'_1 \ldots X'_n) \, Y/D \, E \, Q \, Y \, F \qquad (II)$$
   kodierenden Nucleotidsequenz,
   wobei $X'_1 \ldots X'_n$ eine Sequenz von 5-7 Aminosäuren darstellt,
   (b) einer für die Aminosäuresequenz
   $$C \, A \, (X''_1 \ldots X''_n) \, N \, E \, Q \, F \, F \qquad (III)$$
   kodierenden Nucleotidsequenz,
   wobei $X''_1 \ldots X''_n$ eine Sequenz von 5-7 Aminosäuren darstellt,
   (c) einer für die Aminosäuresequenz
   $$C \, A \, (X'''_1 \ldots X'''_n) \, D \, T \, Q \, Y \, F \qquad (IV)$$
   kodierenden Nucleotidsequenz,
   wobei $X'''_1 \ldots X'''_n$ eine Sequenz von 5-7 Aminosäuren darstellt,
   (d) einer Nucleotidsequenz, die für eine Aminosäuresequenz mit einer Identität von mindestens 80 % zu einer Aminosäuresequenz aus (a), (b) oder/und (c) kodiert, oder
   (e) einer Nucleotidsequenz, die für eine Aminosäuresequenz mit einer äquivalenten Erkennungsspezifität für die Peptidkomponente des T-Zellrezeptor-Liganden kodiert.

9. Nucleinsäure nach Anspruch 8,
   **dadurch gekennzeichnet**,
   daß die Aminosäuresequenz $X'_1 \ldots X'_n$ ausgewählt ist aus der Gruppe, bestehend aus SSETNS, SSETSS, TSGTAS, RSGTGS, SSGTDS, SSGTRS, SSGSDS, SSSTGS, SSSTVS, SSSTLS, SSSTLF, SSSTAS, SSHTDS, SSDTLS und SRWDSE.

10. Vektor,
    **dadurch gekennzeichnet**,
    daß er mindestens eine Kopie einer Nucleinsäure nach einem der Ansprüche 7 bis 9 enthält.

11. Zelle,
    **dadurch gekennzeichnet**,
    daß sie eine Nucleinsäure nach einem der Ansprüche 7 bis 9 exprimiert.

12. Polypeptid,
    **dadurch gekennzeichnet**,
    daß es von einer Nucleinsäure nach einem der Ansprüche 7 bis 9 kodiert ist.

13. Polypeptid,
    **dadurch gekennzeichnet**,
    daß es T-Zellrezeptor-Eigenschaften aufweist und aus einem Polypeptid nach Anspruch 6 sowie einem Polypeptid nach Anspruch 12 als Untereinheiten aufgebaut ist.

14. Polypeptid nach einem der Ansprüche 6, 12 oder 13,
    **dadurch gekennzeichnet**,
    daß es mit einer Markierungsgruppe oder einem Toxin gekoppelt ist.

15. Antikörper gegen ein Polypeptid nach einem der Ansprüche 6, 12, 13 oder 14, der gegen eine für die Erkennung des Peptidliganden verantwortliche Region gerichtet ist.

16. T-Zelle,
    **dadurch gekennzeichnet**,
    daß sie einen T-Zellrezeptor nach Anspruch 13 enthält.

17. Pharmazeutische Zusammensetzung, die als aktive Komponente eine Nucleinsäure nach einem der Ansprüche 1 bis 3 oder 7 bis 9, ein Polypeptid nach einem der Ansprüche 6, 12, 13 oder 14, einen Peptidliganden gegen das Polypeptid, einen Antikörper nach Anspruch 15 oder eine Zelle nach Anspruch 5, 11 oder 16 gegebenenfalls zusammen mit anderen aktiven Komponenten, sowie pharmazeutisch üblichen Hilfs-, Zusatz- oder Trägerstoffen enthält.

18. Verwendung einer pharmazeutischen Zusammensetzung nach Anspruch 17 zur Herstellung eines Mittels für die Diagnose einer Tumorerkrankung oder einer Prädisposition für eine Tumorerkrankung.

19. Verwendung einer pharmazeutischen Zusammensetzung nach Anspruch 17 zur Herstellung eines Mittels für die Überwachung des Krankheitsverlaufs bei einer Tumorerkrankung.

20. Verwendung einer pharmazeutischen Zusammensetzung nach Anspruch 17 zur Herstellung eines Mittels für die Prävention oder Therapie einer Tumorerkrankung.

21. Verfahren zur Gewinnung von T-Zellen, die ein Polypeptid nach Anspruch 13 als T-Zellrezeptor exprimieren,
    **dadurch gekennzeichnet**,
    daß man eine T-Zellen enthaltende Probe mit einem spezifisch an die CDR3-Region des T-Zellrezeptors bindenden Mittel in Kontakt bringt, die mit dem Mittel reagierenden T-Zellen identifiziert und gegebenenfalls von anderen T-Zellen abtrennt.

22. Verfahren zur Gewinnung von T-Zellen, die ein Polypeptid nach Anspruch 13 als T-Zellrezeptor exprimieren,
    **dadurch gekennzeichnet**,
    daß man Nucleinsäuresequenzen, die für den T-Zellrezeptor kodieren, in eine T-Zellinie einführt, und dort zur Expression bringt.

23. Verfahren zur Gewinnung von T-Zellen, die ein Polypeptid nach Anspruch 13 als T-Zellrezeptor exprimieren,
    **dadurch gekennzeichnet**,
    daß man Nucleinsäuresequenzen, die für den T-Zellrezeptor kodieren, in die Keimbahn eines Tieres einführt und die T-Zellen aus dem resultierenden transgenen Tier oder Nachkommen davon gewinnt.

24. Transgenes Tier,
    **dadurch gekennzeichnet**,
    daß es ein Polypeptid nach Anspruch 13 als T-Zellrezeptor exprimiert.

25. Verfahren zur Identifizierung von Peptidliganden eines T-Zellrezeptors nach Anspruch 13, umfassend die Schritte:

    (a) Gewinnen von RNA aus Tumorgewebe,
    (b) Überführen der RNA in doppelsträngige cDNA-Moleküle,
    (c) Einbringen der cDNA-Moleküle in Wirtszellen, wobei eine cDNA-Bank erhalten wird,
    (d) Transfizieren von eukaryontischen Empfängerzellen mit Aliquots der cDNA-Bank, wobei (i) eine Cotransfektion mit HLA-A*0201-DNA erfolgt oder (ii) HLA-A*0201 positive Empfängerzellen verwendet werden,

(e) Testen der transfizierten Empfängerzellen auf Fähigkeit zur Stimulation von T-Zellen,

(f) Identifizieren einer cDNA-Sequenz, die für das Antigen, welches den Peptidliganden enthält, kodiert und

(g) Identifizieren der Sequenz des Peptidliganden.

Abb. 1/1

Ergebnisse    #26 Tumor i.s.    CDR3α-Region

| Fragment | TCRAV20S1 | N-Region | TCRAJ |
|---|---|---|---|
| Klone (14/54) | C  L  V  G<br>TGCCTCGTGGG | TG<br>Va20- oder Ja22-codiert | G  S  A  R  Q  L  T  F<br>GTTCTGCAAGGCAACTGACCTT<br>TCRAJ22 |
| Klone (1/54) | C  L  V  G<br>TGCCTCGTGGG | A | G  S  A  R  Q  L  T  F<br>GGTTCTGCAAGGCAACTGACCTT<br>TCRAJ22 |
| Klone (11/54) | C  L  V<br>TGCCTCGT | L<br>CCT | S  G  S  A  R  Q  L  T  F<br>TTCTGGTTCTGCAAGGCAACTGACCTT<br>TCRAJ22 |
| Klone (5/54) | C  L  V<br>TGCCTCGTG | L<br>CT | S  G  S  A  R  Q  L  T  F<br>TTCTGGTTCTGCAAGGCAACTGACCTT<br>TCRAJ22 |
| Klone (2/54) | C  L<br>TGCCTCG | A<br>CTA | T  G  S  A  R  Q  L  T  F<br>CTGGTTCTTGCAAGGCAACTGACCTT<br>TCRAJ22 |
| Klone (1/54) | C  L<br>TGCCTCG | V<br>T | S  G  S  A  R  Q  L  T  F<br>TTCTGGTTCTGCAAGGCAACTGACCTT<br>TCRAJ22 |
| Klone (1/54) | C  L  V<br>TGCCTCGTGG | V  S<br>TCTCC | G  S  A  R  Q  L  T  F<br>GGTTCTGCAAGGCAACTGACCTT<br>TCRAJ22 |
| Klone (1/54) | C  L<br>TGCCTCG | D  S<br>ACTCC | G  S  A  R  Q  L  T  F<br>GGTTCTGCAAGGCAACTGACCTT<br>TCRAJ22 |
| Klone (1/54) | C  L<br>TGCCTCG | D<br>AC | S  G  S  A  R  Q  L  T  F<br>TCTGGTTCTGCAAGGCAACTGACCTT<br>TCRAJ22 |
| Klone (2/54) | C  L<br>TGCCTCG | A  L  A<br>CCCTGGGG | G  S  A  R  Q  L  T  F<br>GGTTCTGCAAGGCAACTGACCTT<br>TCRAJ22 |

Abb. 1/2

Klone (1/54)      C   L          A   L   A              G   S   A   R   Q   L   T   F
                TGCCTCG          CCCTGGCG              GGTTCTGCAAGGCAACTGACCTT
                                                              TCRAJ22


Klone (1/54)      C   L          A   P          S   G   S   A   R   Q   L   T   F
                TGCCTCG          CGCCC          TCTGGTTCTGCAAGGCAACTGACCTT
                                                              TCRAJ22


Klone (2/54)      C   L                   P   S   G   S   A   R   Q   L   T   F
                TGCCT            TC       CTTCTGGTTCTGCAAGGCAACTGACCTT
                                                              TCRAJ22

Abb. 2/1

Ergebnisse  #26 Tumor i.s.    CDR3β-Region

| Fragment | TCRBV22S1 | N-TCRBD-N | TCRBJ |
|---|---|---|---|

Klone (8/62)  
    C  A  S  S      E   T   N     S   Y   E   Q   Y   F  
    TGTGCCAGCAG     CGAAACTAA    CTCCTACGAGCAGTACTT  
                 TCRBD2                   TCRBJ2S7

Klone (2/62)  
    C  A  S  S      E   T   N     S   Y   E   Q   Y   F  
    TGTGCCAGCAGT    GAAACTAAT    TCCTACGAGCAGTACTT  
                 TCRBD2                   TCRBJ2S7

Klon (1/62)  
    C  A  S  S      E   T   S     S   Y   E   Q   Y   F  
    TGTGCCAGCAGT    GAAACTTCT    TCCTACGAGCAGTACTT  
                 TCRBD2                   TCRBJ2S7

Klon (1/62)  
    C  A  S  S      E   T   S     S   Y   E   Q   Y   F  
    TGTGCCAGCAGT    GAAACAAG    CTCCTACGAGCAGTACTT  
                 TCRBD1                   TCRBJ2S7

---

Klone (2/62)  
    C  A       T   S   G   T   A   S   Y   E   Q   Y   F  
    TGTGCCA    CCTCCGGGACAGCT    TCCTACGAGCAGTACTT  
                 TCRBD1                   TCRBJ2S7

Klone (2/62)  
    C  A  R      S   G   T   G     S   Y   E   Q   Y   F  
    TGTGCCAG    ATCCGGGACAGG    CTCCTACGAGCAGTACTT  
                 TCRBD1                   TCRBJ2S7

Klone (2/62)  
    C  A  S  S      G   T   D     S   Y   E   Q   Y   F  
    TGTGCCAGCAGT    GGGACGGA    CTCCTACGAGCAGTACTT  
                 TCRBD1/2                TCRBJ2S7

Klon (1/62)  
    C  A  S  S      G   T   D     S   Y   E   Q   Y   F  
    TGTGCCAGCAGT    GGCACAGA    CTCCTACGAGCAGTACTT  
                 TCRBD1                   TCRBJ2S7

Klon (1/62)  
    C  A  S  S      G   T   D     S   Y   E   Q   Y   F  
    TGTGCCAGCAG    CGGGACAGAT    TCCTACGAGCAGTACTT  
                 TCRBD1                   TCRBJ2S7

Abb. 2/2

```
Klon (1/62)              C   A   S   S        G   T   R         S   Y   E   Q   Y   F
                         TGTGCCAGCAGT         GGGACTCGT         TCCTACGAGCAGTACTT
                                              TCRBD2                        TCRBJ2S7


Klon (1/62)              C   A   S   S        G   T   R         S   Y   E   Q   Y   F
                         TGTGCCAGCAGT         GGGACACGT         TCCTACGAGCAGTACTT
                                              TCRBD1                        TCRBJ2S7


Klone (2/62)             C   A   S   S       G   T   S   S      Y   N   E   Q   F   F
                         TGTGCCAGCAGT        GGAACTAGCTCTT      ACAATGAGCAGTTCTT
                                              TCRBD2                        TCRBJ2S1
                                                               (TCRBJ2S7 sehr ähnlich)
    :
-----------------------------------------------------------------------------------

Klon (1/62)              C   A   S   S        G   S   D         S   Y   E   Q   Y   F
                         TGTGCCAGCAGT         GGGTCCGA          CTCCTACGAGCAGTACTT
                                              TCRBD1/2                      TCRBJ2S7

_____

Klone (5/62)             C   A   S   S        S   T   G         S   Y   E   Q   Y   F
                         TGTGCCAGCAGT         TCGACAGGG         TCCTACGAGCAGTACTT
                                              TCRBD1                       TCRBJ2S7


Klone (2/62)             C   A   S   S    .   S   T   V         S   Y   E   Q   Y   F
                         TGTGCCAGCAG          CTCGACGGT         CTCCTACGAGCAGTACTT
                                              TCRBD1/2                      TCRBJ2S7


Klon (1/62)              C   A   S   S        S   T   L         S   Y   E   Q   Y   F
                         TGTGCCAGCAGT         TCAACATTA         TCCTACGAGCAGTACTT
                                              TCRBD2                       TCRBJ2S7


Klon (1/62)              C   A   S   S       S   T   L   F          Y   E   Q   Y   F
                         TGTGCCAGCAGT        TCAACATTATT        CTACGAGCAGTACTT
                                              TCRBD2                       TCRBJ2S7


Klon (1/62)              C   A   S   S        S   T   A         S   Y   E   Q   Y   F
                         TGTGCCAGCAGT         TCGACAGC.         CTCCTACGAGCAGTACTT
                                              TCRBD1                       TCRBJ2S7


Klone (2/62)             C   A   S   S        H   T   D         S   Y   E   Q   Y   F
                         TGTGCCAGCAG          CCACACCGA         CTCCTACGAGCAGTACTT
                                              TCRBD1                       TCRBJ2S7


Klone (2/62)             C   A   S   S        D   T   L         S   Y   E   Q   Y   F
                         TGTGCCAGCAGT         GACACCCT          CTCCTACGAGCAGTACTT
                                              TCRBD1                       TCRBJ2S7
```

29

Abb. 3

Ergebnisse  :#22 Tumor i.s.  CDR3α-Region

Klone (13/34)    Y  C  L  V  G                       G  S  A  R  Q  L  T  F
                 TACTGCCTCGTGGG        TG            GTTCTGCAAGGCAACTGACCTT
                              Val6- oder JaC-codiert                TCRAJ22

Klone (2/34)     Y  C  L  V  G                       G  S  A  R  Q  L  T  F
                 TACTGCCTCGTGGG        G             GGTTCTGCAAGGCAACTGACCTT
                                                                   TCRAJ22

Klon (1/34)      Y  C  L  V  G                       R  S  A  R  Q  L  T  F
                 TACTGCCTCGTGGG        TC            GTTCTGCAAGGCAACTGACCTT
                                                                   TCRAJ22

Klone (4/34)     Y  C  L  V            L          S  G  S  A  R  Q  L  T  F
                 TACTGCCTCGT           CCT        TTCTGGTTCTGCAAGGCAACTGACCTT
                                                                   TCRAJ22

Klone (2/34)     Y  C  L               A          T  G  S  A  R  Q  L  T  F
                 TACTGCCTCG            CTA        CTGGTTCTGCAAGGCAACTGACCTT
                                                                   TCRAJ22

30

Abb. 4

Ergebnisse  ;#22 Tumor i.s.     CDR3β-Region

```
Klone (10/28)      C   A   S        A   D   S   F   K        D   T   Q   Y   F
                TGTGCCAG        TGCCGATTCTTTTAA        AGATACGCAGTATTT
                                       TCRBD2                         TCRBJ2S3


Klone (4/28)       C   A   S   S        E   T   N        S   Y   E   Q   Y   F
                TGTGCCAGCAG        CGAAACTAA        CTCCTACGAGCAGTACTT
                                       TCRBD2                            Jb2.7


Klone (1/28)       C   A   S   S        D   Q   G   M        N   E   Q   F   F
                TGTGCCAGCAGT        GATCAGGGGATG        AATGAGCAGTTCTT
                                        TCRBD2                         TCRBJ2S1


Klone (1/28)       C   A   S   R        W   D   S   E        D   E   Q   Y   F
                TGTGCCAGCAG        GTGGGACTCCGAGG        ACGAGCAGTACTT
                                        TCRBD2                         TCRBJ2S7
```

SEQUENZPROTOKOLL

(1) ALLGEMEINE ANGABEN:

    (i) ANMELDER:
        (A) NAME: Boehringer Mannheim GmbH
        (B) STRASSE: Sandhofer Str. 112-132
        (C) ORT: Mannheim
        (E) LAND: Deutschland
        (F) POSTLEITZAHL: 68305

    (ii) BEZEICHNUNG DER ERFINDUNG: Nierenkarzinom-spezifische T-Zellen

  (iii) ANZAHL DER SEQUENZEN: 22

    (iv) COMPUTER-LESBARE FASSUNG:
        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.30 (EPA)

(2) ANGABEN ZU SEQ ID NO: 1:

    (i) SEQUENZKENNZEICHEN:
        (A) LÄNGE: 1341 Basenpaare
        (B) ART: Nucleotid
        (C) STRANGFORM: beides
        (D) TOPOLOGIE: linear

    (ix) MERKMAL:
        (A) NAME/SCHLÜSSEL: CDS
        (B) LAGE:1..801

    (ix) MERKMAL:
        (A) NAME/SCHLÜSSEL: sig_peptide
        (B) LAGE:1..54

    (ix) MERKMAL:
        (A) NAME/SCHLÜSSEL: mat_peptide
        (B) LAGE:55..801

    (xi) SEQUENZBESCHREIBUNG: SEQ ID NO: 1:

```
ATG AGG CAA GTG GCG AGA GTG ATC GTG TTC CTG ACC CTG AGT ACT TTG        48
Met Arg Gln Val Ala Arg Val Ile Val Phe Leu Thr Leu Ser Thr Leu
-18         -15             -10                 -5

AGC CTT GCT AAG ACC ACC CAG CCC ATC TCC ATG GAC TCA TAT GAA GGA        96
Ser Leu Ala Lys Thr Thr Gln Pro Ile Ser Met Asp Ser Tyr Glu Gly
          1               5                   10

CAA GAA GTG AAC ATA ACC TGT AGC CAC AAC AAC ATT GCT ACA AAT GAT       144
Gln Glu Val Asn Ile Thr Cys Ser His Asn Asn Ile Ala Thr Asn Asp
   15                 20                  25                  30

TAT ATC ACG TGG TAC CAA CAG TTT CCC AGC CAA GGA CCA CGA TTT ATT       192
Tyr Ile Thr Trp Tyr Gln Gln Phe Pro Ser Gln Gly Pro Arg Phe Ile
               35                  40                  45
```

```
ATT CAA GGA TAC AAG ACA AAA GTT ACA AAC GAA GTG GCC TCC CTG TTT        240
Ile Gln Gly Tyr Lys Thr Lys Val Thr Asn Glu Val Ala Ser Leu Phe
            50                  55                  60

ATC CCT GCC GAC AGA AAG TCC AGC ACT CTG AGC CTG CCC CGG GTT TCC        288
Ile Pro Ala Asp Arg Lys Ser Ser Thr Leu Ser Leu Pro Arg Val Ser
            65                  70                  75

CTG AGC GAC ACT GCT GTG TAC TAC TGC CTC GTG GGT GGT TCT GCA AGG        336
Leu Ser Asp Thr Ala Val Tyr Tyr Cys Leu Val Gly Gly Ser Ala Arg
        80                  85                  90

CAA CTG ACC TTT GGA TCT GGG ACA CAA TTG ACT GTT TTA CCT GAT ATC        384
Gln Leu Thr Phe Gly Ser Gly Thr Gln Leu Thr Val Leu Pro Asp Ile
95                  100                 105                 110

CAG AAC CCT GAC CCT GCC GTG TAC CAG CTG AGA GAC TCT AAA TCC AGT        432
Gln Asn Pro Asp Pro Ala Val Tyr Gln Leu Arg Asp Ser Lys Ser Ser
                115                 120                 125

GAC AAG TCT GTC TGC CTA TTC ACC GAT TTT GAT TCT CAA ACA AAT GTG        480
Asp Lys Ser Val Cys Leu Phe Thr Asp Phe Asp Ser Gln Thr Asn Val
                130                 135                 140

TCA CAA AGT AAG GAT TCT GAT GTG TAT ATC ACA GAC AAA ACT GTG CTA        528
Ser Gln Ser Lys Asp Ser Asp Val Tyr Ile Thr Asp Lys Thr Val Leu
            145                 150                 155

GAC ATG AGG TCT ATG GAC TTC AAG AGC AAC AGT GCT GTG GCC TGG AGC        576
Asp Met Arg Ser Met Asp Phe Lys Ser Asn Ser Ala Val Ala Trp Ser
        160                 165                 170

AAC AAA TCT GAC TTT GCA TGT GCA AAC GCC TTC AAC AAC AGC ATT ATT        624
Asn Lys Ser Asp Phe Ala Cys Ala Asn Ala Phe Asn Asn Ser Ile Ile
175                 180                 185                 190

CCA GAA GAC ACC TTC TTC CCC AGC CCA GAA AGT TCC TGT GAT GTC AAG        672
Pro Glu Asp Thr Phe Phe Pro Ser Pro Glu Ser Ser Cys Asp Val Lys
                195                 200                 205

CTG GTC GAG AAA AGC TTT GAA ACA GAT ACG AAC CTA AAC TTT CAA AAC        720
Leu Val Glu Lys Ser Phe Glu Thr Asp Thr Asn Leu Asn Phe Gln Asn
            210                 215                 220

CTG TCA GTG ATT GGG TTC CGA ATC CTC CTC CTG AAA GTG GCC GGG TTT        768
Leu Ser Val Ile Gly Phe Arg Ile Leu Leu Leu Lys Val Ala Gly Phe
        225                 230                 235

AAT CTG CTC ATG ACG CTG CGG CTG TGG TCC AGC TGAGATCTGC AAGATTGTAA     821
Asn Leu Leu Met Thr Leu Arg Leu Trp Ser Ser
        240                 245

GACAGCCTGT GCTCCCTCGC TCCTTCCTCT GCATTGCCCC TCTTCTCCCT CTCCAAACAG      881

AGGGAACTCT CCTACCCCCA AGGAGGTGAA AGCTGCTACC ACCTCTGTGC CCCCCCGGCA      941

ATGCCACCAA CTGGATCCTA CCCGAATTTA TGATTAAGAT TGCTGAAGAG CTGCCAAACA     1001

CTGCTGCCAC CCCCTCTGTT CCCTTATTGC TGCTTGTCAC TGCCTGACAT TCACGGCAGA     1061

GGCAAGGCTG CTGCAGCCTC CCCTGGCTGT GCACATTCCC TCCTGCTCCC CAGAGACTGC     1121

CTCCGCCATC CCACAGATGA TGGATCTTCA GTGGGTTCTC TTGGGCTCTA GGTCCTGGAG     1181
```

13

```
AATGTTGTGA GGGGTTTATT TTTTTTTAAT AGTGTTCATA AAGAAATACA TAGTATTCTT    1241

CTTCTCAAGA CGTGGGGGGA AATTATCTCA TTATCGAGGC CCTGCTATGC TGTGTGTCTG    1301

GGCGTGTTGT ATGTCCTGCT GCCGATGCCT TCATTAAAAT                          1341
```

(2) ANGABEN ZU SEQ ID NO: 2:

   (i) SEQUENZKENNZEICHEN:
    (A) LÄNGE: 267 Aminosäuren
    (B) ART: Aminosäure
    (D) TOPOLOGIE: linear

   (ii) ART DES MOLEKÜLS: Protein
   (xi) SEQUENZBESCHREIBUNG: SEQ ID NO: 2:

```
Met Arg Gln Val Ala Arg Val Ile Val Phe Leu Thr Leu Ser Thr Leu
-18          -15                 -10                 -5

Ser Leu Ala Lys Thr Thr Gln Pro Ile Ser Met Asp Ser Tyr Glu Gly
             1               5               10

Gln Glu Val Asn Ile Thr Cys Ser His Asn Asn Ile Ala Thr Asn Asp
    15               20               25                       30

Tyr Ile Thr Trp Tyr Gln Gln Phe Pro Ser Gln Gly Pro Arg Phe Ile
                 35               40                       45

Ile Gln Gly Tyr Lys Thr Lys Val Thr Asn Glu Val Ala Ser Leu Phe
             50               55                       60

Ile Pro Ala Asp Arg Lys Ser Ser Thr Leu Ser Leu Pro Arg Val Ser
         65               70               75

Leu Ser Asp Thr Ala Val Tyr Tyr Cys Leu Val Gly Gly Ser Ala Arg
     80               85               90

Gln Leu Thr Phe Gly Ser Gly Thr Gln Leu Thr Val Leu Pro Asp Ile
 95               100              105                      110

Gln Asn Pro Asp Pro Ala Val Tyr Gln Leu Arg Asp Ser Lys Ser Ser
             115              120                      125

Asp Lys Ser Val Cys Leu Phe Thr Asp Phe Asp Ser Gln Thr Asn Val
             130              135              140

Ser Gln Ser Lys Asp Ser Asp Val Tyr Ile Thr Asp Lys Thr Val Leu
         145              150              155

Asp Met Arg Ser Met Asp Phe Lys Ser Asn Ser Ala Val Ala Trp Ser
    160              165              170

Asn Lys Ser Asp Phe Ala Cys Ala Asn Ala Phe Asn Asn Ser Ile Ile
175              180              185                      190

Pro Glu Asp Thr Phe Phe Pro Ser Pro Glu Ser Ser Cys Asp Val Lys
             195              200              205

Leu Val Glu Lys Ser Phe Glu Thr Asp Thr Asn Leu Asn Phe Gln Asn
             210              215                      220

Leu Ser Val Ile Gly Phe Arg Ile Leu Leu Leu Lys Val Ala Gly Phe
         225              230              235
```

```
Asn Leu Leu Met Thr Leu Arg Leu Trp Ser Ser
    240                 245
```

(2) ANGABEN ZU SEQ ID NO: 3:

    (i) SEQUENZKENNZEICHEN:
        (A) LÄNGE: 936 Basenpaare
        (B) ART: Nucleotid
        (C) STRANGFORM: beides
        (D) TOPOLOGIE: linear

    (ix) MERKMAL:
        (A) NAME/SCHLÜSSEL: CDS
        (B) LAGE:1..933

    (ix) MERKMAL:
        (A) NAME/SCHLÜSSEL: sig_peptide
        (B) LAGE:1..63

    (ix) MERKMAL:
        (A) NAME/SCHLÜSSEL: mat_peptide
        (B) LAGE:64..933

    (xi) SEQUENZBESCHREIBUNG: SEQ ID NO: 3:

```
ATG GAT ACC TGG CTC GTA TGC TGG GCA ATT TTT AGT CTC TTG AAA GCA        48
Met Asp Thr Trp Leu Val Cys Trp Ala Ile Phe Ser Leu Leu Lys Ala
-21 -20             -15                 -10

GGA CTC ACA GAA CCT GAA GTC ACC CAG ACT CCC AGC CAT CAG GTC ACA        96
Gly Leu Thr Glu Pro Glu Val Thr Gln Thr Pro Ser His Gln Val Thr
 -5             1               5                   10

CAG ATG GGA CAG GAA GTG ATC TTG CGC TGT GTC CCC ATC TCT AAT CAC       144
Gln Met Gly Gln Glu Val Ile Leu Arg Cys Val Pro Ile Ser Asn His
            15                  20                  25

TTA TAC TTC TAT TGG TAC AGA CAA ATC TTG GGG CAG AAA GTC GAG TTT       192
Leu Tyr Phe Tyr Trp Tyr Arg Gln Ile Leu Gly Gln Lys Val Glu Phe
            30                  35                  40

CTG GTT TCC TTT TAT AAT AAT GAA ATC TCA GAG AAG TCT GAA ATA TTC       240
Leu Val Ser Phe Tyr Asn Asn Glu Ile Ser Glu Lys Ser Glu Ile Phe
        45                  50                  55

GAT GAT CAA TTC TCA GTT GAA AGG CCT GAT GGA TCA AAT TTC ACT CTG       288
Asp Asp Gln Phe Ser Val Glu Arg Pro Asp Gly Ser Asn Phe Thr Leu
    60                  65                  70                  75

AAG ATC CGG TCC ACA AAG CTG GAG GAC TCA GCC ATG TAC TTC TGT GCC       336
Lys Ile Arg Ser Thr Lys Leu Glu Asp Ser Ala Met Tyr Phe Cys Ala
                80                  85                  90

AGC AGC GAA ACT AAC TCC TAC GAG CAG TAC TTC GGG CCG GGC ACC AGG       384
Ser Ser Glu Thr Asn Ser Tyr Glu Gln Tyr Phe Gly Pro Gly Thr Arg
                95                  100                 105

CTC ACG GTC ACA GAG GAC CTG AAA AAC GTG TTC CCA CCC GAG GTC GCT       432
Leu Thr Val Thr Glu Asp Leu Lys Asn Val Phe Pro Pro Glu Val Ala
        110                 115                 120
```

```
GTG TTT GAG CCA TCA GAA GCA GAG ATC TCC CAC ACC CAA AAG GCC ACA      480
Val Phe Glu Pro Ser Glu Ala Glu Ile Ser His Thr Gln Lys Ala Thr
    125                 130                 135

CTG GTG TGC CTG GCC ACA GGC TTC TAC CCC GAC CAC GTG GAG CTG AGC      528
Leu Val Cys Leu Ala Thr Gly Phe Tyr Pro Asp His Val Glu Leu Ser
140                 145                 150                 155

TGG TGG GTG AAT GGG AAG GAG GTG CAC AGT GGG GTC AGC ACA GAC CCG      576
Trp Trp Val Asn Gly Lys Glu Val His Ser Gly Val Ser Thr Asp Pro
                160                 165                 170

CAG CCC CTC AAG GAG CAG CCC GCC CTC AAT GAC TCC AGA TAC TGC CTG      624
Gln Pro Leu Lys Glu Gln Pro Ala Leu Asn Asp Ser Arg Tyr Cys Leu
                175                 180                 185

AGC AGC CGC CTG AGG GTC TCG GCC ACC TTC TGG CAG AAC CCC CGC AAC      672
Ser Ser Arg Leu Arg Val Ser Ala Thr Phe Trp Gln Asn Pro Arg Asn
            190                 195                 200

CAC TTC CGC TGT CAA GTC CAG TTC TAC GGG CTC TCG GAG AAT GAC GAG      720
His Phe Arg Cys Gln Val Gln Phe Tyr Gly Leu Ser Glu Asn Asp Glu
    205                 210                 215

TGG ACC CAG GAT AGG GCC AAA CCT GTC ACC CAG ATC GTC AGC GCC GAG      768
Trp Thr Gln Asp Arg Ala Lys Pro Val Thr Gln Ile Val Ser Ala Glu
220                 225                 230                 235

GCC TGG GGT AGA GCA GAC TGT GGC TTC ACC TCC GAG TCT TAC CAG CAA      816
Ala Trp Gly Arg Ala Asp Cys Gly Phe Thr Ser Glu Ser Tyr Gln Gln
                240                 245                 250

GGG GTC CTG TCT GCC ACC ATC CTC TAT GAG ATC TTG CTA GGG AAG GCC      864
Gly Val Leu Ser Ala Thr Ile Leu Tyr Glu Ile Leu Leu Gly Lys Ala
                255                 260                 265

ACC TTG TAT GCC GTG CTG GTC AGT GCC CTC GTG CTG ATG GCC ATG GTC      912
Thr Leu Tyr Ala Val Leu Val Ser Ala Leu Val Leu Met Ala Met Val
                270                 275                 280

AAG AGA AAG GAT TCC AGA GGC TAG                                      936
Lys Arg Lys Asp Ser Arg Gly
    285                 290
```

(2) ANGABEN ZU SEQ ID NO: 4:

      (i) SEQUENZKENNZEICHEN:
        (A) LÄNGE: 311 Aminosäuren
        (B) ART: Aminosäure
        (D) TOPOLOGIE: linear

    (ii) ART DES MOLEKÜLS: Protein
    (xi) SEQUENZBESCHREIBUNG: SEQ ID NO: 4:

```
Met Asp Thr Trp Leu Val Cys Trp Ala Ile Phe Ser Leu Leu Lys Ala
-21 -20                 -15                 -10

Gly Leu Thr Glu Pro Glu Val Thr Gln Thr Pro Ser His Gln Val Thr
 -5                  1                  5                 10

Gln Met Gly Gln Glu Val Ile Leu Arg Cys Val Pro Ile Ser Asn His
                15                  20                 25
```

16

```
Leu Tyr Phe Tyr Trp Tyr Arg Gln Ile Leu Gly Gln Lys Val Glu Phe
        30                      35                  40

Leu Val Ser Phe Tyr Asn Asn Glu Ile Ser Glu Lys Ser Glu Ile Phe
        45                  50                  55

Asp Asp Gln Phe Ser Val Glu Arg Pro Asp Gly Ser Asn Phe Thr Leu
60                      65                  70                  75

Lys Ile Arg Ser Thr Lys Leu Glu Asp Ser Ala Met Tyr Phe Cys Ala
                80                      85                  90

Ser Ser Glu Thr Asn Ser Tyr Glu Gln Tyr Phe Gly Pro Gly Thr Arg
                95                      100                 105

Leu Thr Val Thr Glu Asp Leu Lys Asn Val Phe Pro Pro Glu Val Ala
        110                     115                 120

Val Phe Glu Pro Ser Glu Ala Glu Ile Ser His Thr Gln Lys Ala Thr
        125                     130                 135

Leu Val Cys Leu Ala Thr Gly Phe Tyr Pro Asp His Val Glu Leu Ser
140                     145                 150                 155

Trp Trp Val Asn Gly Lys Glu Val His Ser Gly Val Ser Thr Asp Pro
                160                     165                 170

Gln Pro Leu Lys Glu Gln Pro Ala Leu Asn Asp Ser Arg Tyr Cys Leu
                175                     180                 185

Ser Ser Arg Leu Arg Val Ser Ala Thr Phe Trp Gln Asn Pro Arg Asn
            190                     195                 200

His Phe Arg Cys Gln Val Gln Phe Tyr Gly Leu Ser Glu Asn Asp Glu
        205                     210                 215

Trp Thr Gln Asp Arg Ala Lys Pro Val Thr Gln Ile Val Ser Ala Glu
220                     225                 230                 235

Ala Trp Gly Arg Ala Asp Cys Gly Phe Thr Ser Glu Ser Tyr Gln Gln
                240                     245                 250

Gly Val Leu Ser Ala Thr Ile Leu Tyr Glu Ile Leu Leu Gly Lys Ala
            255                     260                 265

Thr Leu Tyr Ala Val Leu Val Ser Ala Leu Val Leu Met Ala Met Val
        270                     275                 280

Lys Arg Lys Asp Ser Arg Gly
    285                 290
```

(2) ANGABEN ZU SEQ ID NO: 5:

    (i) SEQUENZKENNZEICHEN:
        (A) LÄNGE: 39 Basenpaare
        (B) ART: Nucleotid
        (C) STRANGFORM: beides
        (D) TOPOLOGIE: linear

    (ix) MERKMAL:
        (A) NAME/SCHLÜSSEL: CDS

(B) LAGE:1..39

(xi) SEQUENZBESCHREIBUNG: SEQ ID NO: 5:

```
TGC CTC GTC CTT TCT GGT TCT GCA AGG CAA CTG ACC TTT                39
Cys Leu Val Leu Ser Gly Ser Ala Arg Gln Leu Thr Phe
            295                 300
```

(2) ANGABEN ZU SEQ ID NO: 6:

        (i) SEQUENZKENNZEICHEN:
            (A) LÄNGE: 13 Aminosäuren
            (B) ART: Aminosäure
            (D) TOPOLOGIE: linear

    (ii) ART DES MOLEKÜLS: Protein
    (xi) SEQUENZBESCHREIBUNG: SEQ ID NO: 6:

```
Cys Leu Val Leu Ser Gly Ser Ala Arg Gln Leu Thr Phe
  1               5                 10
```

(2) ANGABEN ZU SEQ ID NO: 7:

        (i) SEQUENZKENNZEICHEN:
            (A) LÄNGE: 36 Basenpaare
            (B) ART: Nucleotid
            (C) STRANGFORM: beides
            (D) TOPOLOGIE: linear

        (ix) MERKMAL:
            (A) NAME/SCHLÜSSEL: CDS
            (B) LAGE:1..36

        (xi) SEQUENZBESCHREIBUNG: SEQ ID NO: 7:

```
TGC CTC GCT ACT GGT TCT GCA AGG CAA CTG ACC TTT                    36
Cys Leu Ala Thr Gly Ser Ala Arg Gln Leu Thr Phe
        15                  20                  25
```

(2) ANGABEN ZU SEQ ID NO: 8:

        (i) SEQUENZKENNZEICHEN:
            (A) LÄNGE: 12 Aminosäuren
            (B) ART: Aminosäure
            (D) TOPOLOGIE: linear

    (ii) ART DES MOLEKÜLS: Protein
    (xi) SEQUENZBESCHREIBUNG: SEQ ID NO: 8:

```
Cys Leu Ala Thr Gly Ser Ala Arg Gln Leu Thr Phe
  1               5                 10
```

(2) ANGABEN ZU SEQ ID NO: 9:

        (i) SEQUENZKENNZEICHEN:
            (A) LÄNGE: 39 Basenpaare
            (B) ART: Nucleotid
            (C) STRANGFORM: beides

(D) TOPOLOGIE: linear


(ix) MERKMAL:
    (A) NAME/SCHLÜSSEL: CDS
    (B) LAGE:1..39


(xi) SEQUENZBESCHREIBUNG: SEQ ID NO: 9:

```
TGT GCC AGC AGT GGA ACA GAT TCC TAC GAG CAG TAC TTC                    39
Cys Ala Ser Ser Gly Thr Asp Ser Tyr Glu Gln Tyr Phe
        15                  20                  25
```


(2) ANGABEN ZU SEQ ID NO: 10:

        (i) SEQUENZKENNZEICHEN:
            (A) LÄNGE: 13 Aminosäuren
            (B) ART: Aminosäure
            (D) TOPOLOGIE: linear

        (ii) ART DES MOLEKÜLS: Protein
(xi) SEQUENZBESCHREIBUNG: SEQ ID NO: 10:

```
Cys Ala Ser Ser Gly Thr Asp Ser Tyr Glu Gln Tyr Phe
 1               5                  10
```

(2) ANGABEN ZU SEQ ID NO: 11:

        (i) SEQUENZKENNZEICHEN:
            (A) LÄNGE: 39 Basenpaare
            (B) ART: Nucleotid
            (C) STRANGFORM: beides
            (D) TOPOLOGIE: linear


        (ix) MERKMAL:
            (A) NAME/SCHLÜSSEL: CDS
            (B) LAGE:1..39


(xi) SEQUENZBESCHREIBUNG: SEQ ID NO: 11:

```
TGT GCC AGC AGT GAA ACA GAT TCC TAC GAG CAG TAC TTC                    39
Cys Ala Ser Ser Glu Thr Asp Ser Tyr Glu Gln Tyr Phe
        15                  20                  25
```


(2) ANGABEN ZU SEQ ID NO: 12:

        (i) SEQUENZKENNZEICHEN:
            (A) LÄNGE: 13 Aminosäuren
            (B) ART: Aminosäure
            (D) TOPOLOGIE: linear

        (ii) ART DES MOLEKÜLS: Protein
(xi) SEQUENZBESCHREIBUNG: SEQ ID NO: 12:

```
Cys Ala Ser Ser Glu Thr Asp Ser Tyr Glu Gln Tyr Phe
 1               5                  10
```

(2) ANGABEN ZU SEQ ID NO: 13:

    (i) SEQUENZKENNZEICHEN:
        (A) LÄNGE: 39 Basenpaare
        (B) ART: Nucleotid
        (C) STRANGFORM: beides
        (D) TOPOLOGIE: linear


    (ix) MERKMAL:
        (A) NAME/SCHLÜSSEL: CDS
        (B) LAGE:1..39


    (xi) SEQUENZBESCHREIBUNG: SEQ ID NO: 13:

```
TGT GCC AGC AGT GGA ACA GCT TCC TAC GAG CAG TAC TTC          39
Cys Ala Ser Ser Gly Thr Ala Ser Tyr Glu Gln Tyr Phe
    15                  20                  25
```

(2) ANGABEN ZU SEQ ID NO: 14:

    (i) SEQUENZKENNZEICHEN:
        (A) LÄNGE: 13 Aminosäuren
        (B) ART: Aminosäure
        (D) TOPOLOGIE: linear

    (ii) ART DES MOLEKÜLS: Protein
    (xi) SEQUENZBESCHREIBUNG: SEQ ID NO: 14:

```
Cys Ala Ser Ser Gly Thr Ala Ser Tyr Glu Gln Tyr Phe
 1               5                  10
```

(2) ANGABEN ZU SEQ ID NO: 15:

    (i) SEQUENZKENNZEICHEN:
        (A) LÄNGE: 39 Basenpaare
        (B) ART: Nucleotid
        (C) STRANGFORM: beides
        (D) TOPOLOGIE: linear


    (ix) MERKMAL:
        (A) NAME/SCHLÜSSEL: CDS
        (B) LAGE:1..39


    (xi) SEQUENZBESCHREIBUNG: SEQ ID NO: 15:

```
TGT GCC AGC AGT GGT ACA AAC TCC TAC GAG CAG TAC TTT          39
Cys Ala Ser Ser Gly Thr Asn Ser Tyr Glu Gln Tyr Phe
    15                  20                  25
```

(2) ANGABEN ZU SEQ ID NO: 16:

    (i) SEQUENZKENNZEICHEN:
        (A) LÄNGE: 13 Aminosäuren
        (B) ART: Aminosäure
        (D) TOPOLOGIE: linear

```
          (ii) ART DES MOLEKÜLS: Protein
          (xi) SEQUENZBESCHREIBUNG: SEQ ID NO: 16:

Cys Ala Ser Ser Gly Thr Asn Ser Tyr Glu Gln Tyr Phe
  1               5                   10

(2) ANGABEN ZU SEQ ID NO: 17:

          (i) SEQUENZKENNZEICHEN:
                (A) LÄNGE: 39 Basenpaare
                (B) ART: Nucleotid
                (C) STRANGFORM: beides
                (D) TOPOLOGIE: linear



          (ix) MERKMAL:
                (A) NAME/SCHLÜSSEL: CDS
                (B) LAGE:1..39


          (xi) SEQUENZBESCHREIBUNG: SEQ ID NO: 17:

TGT GCC ACC TCC GGG ACA GCT TCC TAC GAG CAG TAC TTC              39
Cys Ala Thr Ser Gly Thr Ala Ser Tyr Glu Gln Tyr Phe
  15              20                  25


(2) ANGABEN ZU SEQ ID NO: 18:

          (i) SEQUENZKENNZEICHEN:
                (A) LÄNGE: 13 Aminosäuren
                (B) ART: Aminosäure
                (D) TOPOLOGIE: linear

          (ii) ART DES MOLEKÜLS: Protein
          (xi) SEQUENZBESCHREIBUNG: SEQ ID NO: 18:

Cys Ala Thr Ser Gly Thr Ala Ser Tyr Glu Gln Tyr Phe
  1               5                   10

(2) ANGABEN ZU SEQ ID NO: 19:

          (i) SEQUENZKENNZEICHEN:
                (A) LÄNGE: 39 Basenpaare
                (B) ART: Nucleotid
                (C) STRANGFORM: beides
                (D) TOPOLOGIE: linear


          (ix) MERKMAL:
                (A) NAME/SCHLÜSSEL: CDS
                (B) LAGE:1..39


          (xi) SEQUENZBESCHREIBUNG: SEQ ID NO: 19:

TGT GCC AGA TCC GGG ACA GGC TCC TAC GAG CAG TAC TTC              39
Cys Ala Arg Ser Gly Thr Gly Ser Tyr Glu Gln Tyr Phe
  15              20                  25


(2) ANGABEN ZU SEQ ID NO: 20:
```

```
    (i) SEQUENZKENNZEICHEN:
        (A) LÄNGE: 13 Aminosäuren
        (B) ART: Aminosäure
        (D) TOPOLOGIE: linear

   (ii) ART DES MOLEKÜLS: Protein
   (xi) SEQUENZBESCHREIBUNG: SEQ ID NO: 20:

Cys Ala Arg Ser Gly Thr Gly Ser Tyr Glu Gln Tyr Phe
 1               5                   10

(2) ANGABEN ZU SEQ ID NO: 21:

    (i) SEQUENZKENNZEICHEN:
        (A) LÄNGE: 20 Basenpaare
        (B) ART: Nucleotid
        (C) STRANGFORM: Einzelstrang
        (D) TOPOLOGIE: linear




   (xi) SEQUENZBESCHREIBUNG: SEQ ID NO: 21:

CACTGAAGAT CCATCATCTG                                          20

(2) ANGABEN ZU SEQ ID NO: 22:

    (i) SEQUENZKENNZEICHEN:
        (A) LÄNGE: 20 Basenpaare
        (B) ART: Nucleotid
        (C) STRANGFORM: Einzelstrang
        (D) TOPOLOGIE: linear




   (xi) SEQUENZBESCHREIBUNG: SEQ ID NO: 22:

TAGAGGATGG TGGCAGACAG                                          20
```